# EUROPEAN PATENT APPLICATION

(11) **EP 4 603 073 A1**
(43) Date of publication of application: **20.08.2025**
(21) Application number: 24158213.9
(22) Date of filing: 16.02.2024
(51) Int. Cl.: A61F 13/551

(54) **PACKAGES OF FOLDED ABSORBENT ARTICLES AND FOLDED ABSORBENT ARTICLES**

(71) Applicant: The Procter & Gamble Company, Cincinnati, OH 45202 (US)
(72) Inventor: ROSAR, Markus, 65824 Schwalbach am Taunus (DE); RINNERT, Torsten, 65824 Schwalbach am Taunus (DE); SANTOS, Teresa, 65824 Schwalbach am Taunus (DE); UNGER, Alexander Eberhard, 65824 Schwalbach am Taunus (DE); FRINGS, Frank Hubert, 53881 Euskirchen (DE); WEISMAN, Paul Thomas, Cincinnati, 45202 (US); KING, Scott Alan, Cincinnati, 45202 (US)
(74) Representative: P&G Patent Germany

(57) **Abstract**

A package (1) comprising a plurality of absorbent articles (10) such as diapers comprising a flexible package material. The articles are folded along a first fold line (400) , which is substantially perpendicular to a central longitudinal axis (50) and a second fold line (500) extending transversely across a front and back waist regions (12, 16) along a second fold line, which is substantially perpendicular to the central longitudinal axis. The package may have a Package Out-of-Stack Compression Deflection Differential at 0.6 psi of at most 3.0 mm according to the Package Compression Test detailed herein. The second fold line may lie between the first fold line (400) and a front waist edge (112) of the absorbent core in the front waist region and/ or lies between the first fold line and a back waist edge (116) of the absorbent core in the back waist region.

## Description

### FIELD OF THE INVENTION

The invention further relates to a flexible package comprising folded absorbent articles, such as diapers and the like. The invention further relates to folded absorbent articles.

### BACKGROUND OF THE INVENTION

Absorbent articles such as diapers and the like are frequently piled into a stack which is compressed before packaging. For transport and warehousing, a plurality of packages of absorbent articles are stacked on pallets. In turn, pallets with a plurality of absorbent articles may again be stacked to optimize storage exploitation, e.g. in transport containers. For transport efficiency, both the dimensions of the absorbent article packages, thus the density of absorbent articles within the packages, and stability of the packages, the stacks of packages and pallets comprising the stacks of packages, thus increased stackability, are crucial. Increased stability, i.e. pressure resistance, of packages, stacks of packages and pallets comprising stacks of packages further improves safety during transport.

In addition, the uniform stability of packages is crucial for placing - and typically again stacking - them on shelves in retail stores. If the package is - partly - deformed during transport, on-shelf stability and thus display appearance and consumer safety may be compromised. Moreover, increasing the absorbent article density within the package and/ or the stackability of packages and/ or pallets of packages leads to material savings: Both packaging material and pallet wrapping material, utilized for stabilization and protection of the pallets, can be reduced further contributing to an improved environmental footprint.

Typically, absorbent articles such as diapers are generally non-uniform in their cross-section resulting in uneven distribution of the compression force within the package, in turn leading to potential instability in stacks of packages and or pallets during transport. There have been approaches to optimize absorbent article density within the package.

Net, there is still need for improvements to enable optimal space exploitation within shipping units and warehousing, less packaging material to be required to overall reduce the environmental footprint of absorbent article packages. In addition, increased safety and reduced damaging risk to packages due to increased stability of packages, in particular on shelf, and pallets comprising a plurality of packages should be achieved.

### SUMMARY OF THE INVENTION

Packages comprising a plurality of folded absorbent articles in the form of diapers and/ or pants having a defined and uniform pressure response and folded absorbent articles having at least two specific folds are provided. Due to such packages comprising folded diapers, the environmental footprint of absorbent article packages is reduced due to enhanced transport efficiency and decreased packaging material consumption. Additionally, the stability and stackability of the packages is increased.

A package comprising a plurality of absorbent articles (10) in the form of taped diapers is provided. The package comprises a flexible package material. Each of the plurality of absorbent articles is in open form. The absorbent articles each comprise a liquid permeable topsheet,a liquid impermeable backsheet, and an absorbent core. The core is at least partially positioned between the topsheet and the backsheet; The absorbent articles each further comprise a front waist region, a back waist region, a crotch region located between the front waist region and the back waist region, and a central longitudinal axis. Each absorbent article exhibits a first fold extending transversely across the crotch region along a first fold line, which is substantially perpendicular to the central longitudinal axis; and further comprise a second fold extending transversely across the front and back waist regions along a second fold line, which is substantially perpendicular to the central longitudinal axis. The package has a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm according to the Package Compression Test detailed herein. Due to such Package Out-of-Stack Compression Deflection Differential represents the packages exhibit a more uniform pressure response and increased stability during transport, warehousing and on shelf.

Further an absorbent article in the form of taped diapers is provided. The article comprises a liquid permeable topsheet, a liquid impermeable backsheet. The core is at least partially positioned between the topsheet and the backsheet; The absorbent article further comprises a front waist region, a back waist region, a crotch region located between the front waist region and the back waist region, and a central longitudinal axis. The absorbent article exhibits a first fold extending transversely across the crotch region along a first fold line, which is substantially perpendicular to the central longitudinal axis; and further comprise a second fold extending transversely across the front and back waist regions along a second fold line, which is substantially perpendicular to the central longitudinal axis. The second fold line lies between the first fold line and a front waist edge of the absorbent core in the front waist region and/ or lies between the first fold line and a back waist edge of the absorbent core in the back waist region. By such folding of an absorbent article with a more uniform pressure response is provided.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1 is a plan view of an example absorbent article in the form of a taped diaper, garment-facing surface facing the viewer, in a flat laid-out state;
Fig. 2 is a plan view of the example absorbent article of Fig. 1, wearer-facing surface facing the viewer, in a flat laid-out state;
Fig. 3 is a front perspective view of the absorbent article of Figs. 1 and 2 in a fastened position;
Fig. 4 is a front perspective view of an absorbent article in the form of a pant;
Fig. 5 is a rear perspective view of the absorbent article of Fig. 4;
Fig. 6 is a plan view of the absorbent article of Fig. 4, laid flat, with a garment-facing surface facing the viewer;
Fig. 7 is a cross-sectional view of the absorbent article taken about line 7-7 of Fig. 6;
Fig. 8 is a cross-sectional view of the absorbent article taken about line 8-8 of Fig. 6;
Fig. 9 is a plan view of an example absorbent core or an absorbent article;
Fig. 10 is a cross-sectional view, taken about line 10-10, of the absorbent core of Fig. 9;
Fig. 11 is a cross-sectional view, taken about line 11-11, of the absorbent core of Fig. 9.

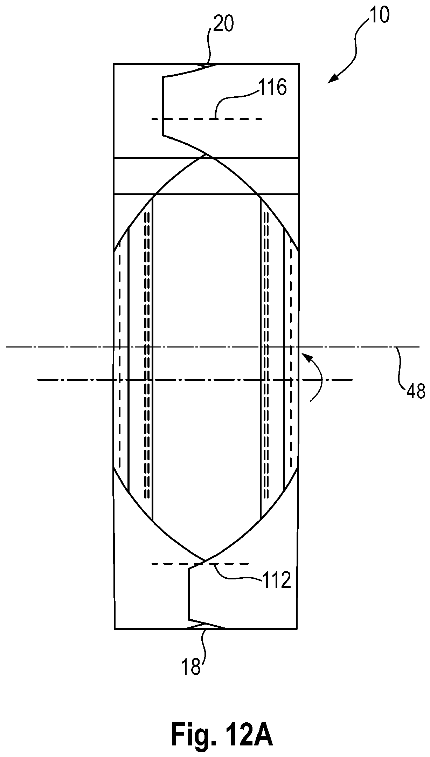

Fig. 12a is a plan view of a taped diaper in open form with side portions folded over and laterally inward about longitudinal side edge fold lines.

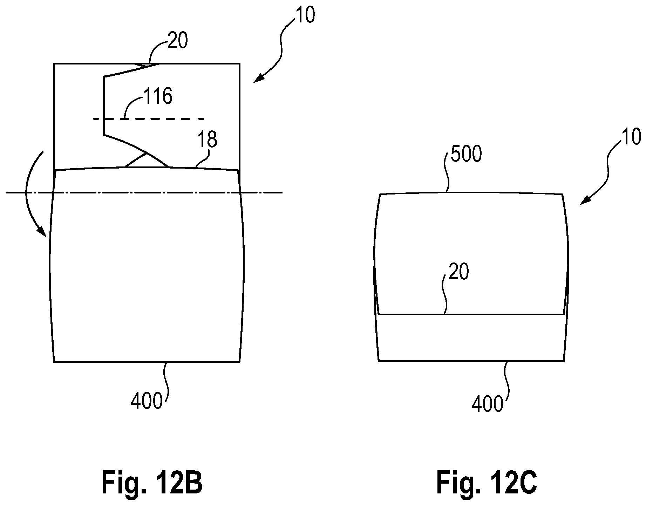

Fig. 12b is a plan view of the diaper of Fig. 12a shown folded about a first fold line.
Fig. 12c is a plan view of the diaper of Fig. 12b shown additionally folded about a second fold line.

### DETAILED DESCRIPTION

Various non-limiting forms of the present disclosure will now be described to provide an overall understanding of the principles of the structure, function, manufacture, and use of the packages of folded absorbent articles and folded absorbent articles disclosed herein. One or more examples of these non-limiting forms are illustrated in the accompanying drawings. Those of ordinary skill in the art will understand that the packages of folded absorbent articles and folded absorbent articles described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

### Definitions

"Absorbent article" refers to devices that absorb and contain body exudates, particularly urine and other water-containing liquids, and, more specifically, refers to devices that are placed against or in proximity to the body of the wearer to absorb and contain the various exudates discharged from the body. Absorbent articles include taped diapers (diapers for babies and infants and diapers to address adult incontinence), and pants (pants for babies and infants and pants to address adult incontinence). Also, preferred absorbent articles of the present invention are disposable absorbent articles, more preferably disposable diapers and disposable pants.

"Absorbent core" is used herein to refer to a structure intended to be disposed between a topsheet and backsheet of an absorbent article for absorbing and storing liquid received by the absorbent article.

"Airfelt" is used herein to refer to comminuted wood pulp, which is a form of cellulosic fiber.

"Closed form" means opposing waist regions are joined to form a continuous waist opening and leg openings as presented to the consumer in a package. An example of closed form is pants. "Open form" means that opposing waist regions are not joined as presented to the consumer in a package. An example of open form is taped diapers. Hence, in an open form the waist opening and leg openings are only formed when the diaper is applied onto a wearer by (releasable) attaching the longitudinal edges of the first and second waist region to each other on both sides by a suitable fastening system.

The terms "compression" or "compressed" as used herein are intended to mean the reduction in volume when a predetermined force is applied to an absorbent article or to a stack of absorbent articles. This reduction in volume may be between 20% and 70% of the uncompressed volume.

"Disposable" is used in its ordinary sense to mean an article that is disposed or discarded after a limited number of usage events over varying lengths of time, for example, less than 10 events, less than 5 events, or less than 2 events. If the disposable absorbent article is a diaper, a pant, absorbent insert, sanitary napkin, sanitary pad or wet wipe for personal hygiene use, the disposable absorbent article is most often intended to be disposed after single use. The used and disposed absorbent article may or may not be subsequently recycled.

"Diaper" and "pant" refers to an absorbent article generally worn by babies, infants and incontinent persons about the lower torso so as to encircle the waist and legs of the wearer and that is specifically adapted to receive and contain urinary and fecal waste. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be pre-formed anywhere along the circumference of the article (e.g., side fastened, front waist fastened). In a diaper (e.g., taped diaper), the waist opening and leg openings are only formed when the diaper is applied onto a wearer by (releasable) attaching the longitudinal edges of the first and second waist region to each other on both sides by a suitable fastening system.

The term "taped diaper" (also referred to as "open diaper") refers to disposable absorbent articles having an initial front waist region and an initial back waist region that are not fastened, pre-fastened, or connected to each other as packaged, prior to being applied to the wearer. A taped diaper may be folded about the lateral centerline with the interior of one waist region in surface to surface contact with the interior of the opposing waist region without fastening or joining the waist regions together. Example taped diapers are disclosed in various suitable configurations are disclosed in U.S. Pat. Nos. 5,167,897, 5,360,420, 5,599,335, 5,643,588, 5,674,216, 5,702,551, 5,968,025, 6,107,537, 6,118,041, 6,153,209, 6,410,129, 6,426,444, 6,586,652, 6,627,787, 6,617,016, 6,825,393, and 6,861,571.

The term "pant" (also referred to as "training pant", "pre-closed diaper", "diaper-pant", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, rear waist fastened or seamed). Example diaper pants in various configurations are disclosed in U.S. Pat. Nos. 5,246,433; 5,569,234; 6,120,487; 6,120,489; 4,940,464; 5,092,861; 5,897,545; 5,957,908; and U.S. Patent Publication No. 2003/0233082.

"Superabsorbent polymer material" ("SAP material") is used herein to refer to crosslinked polymeric materials that can absorb at least 7 times their weight of an aqueous 0.9 weight-% saline solution as measured using the Centrifuge Retention Capacity test set out below. Superabsorbent polymer material of the present invention contains polymers that comprise as monomer groups acrylic acid and/or acrylate and/or methacrylic acid and/or methacrylate. The SAP material is preferably provided in the form of superabsorbent polymer particles (SAP particles) and/or in the form of superabsorbent fibers (SAF).

"Superabsorbent polymer particles" ("SAP particles") is used herein to refer to superabsorbent polymer material that is in particulate form so as to be flowable in the dry state. Superabsorbent polymer particles are distinguished from the superabsorbent fibers of the present invention in that their ratio of largest to smallest dimension is not more than 10 to 1. Superabsorbent polymer particles may for example be in the form of granules, spheres, flakes or agglomerates.

As used herein, the term "nonwoven web" refers to a material which is a manufactured web/layer of directionally or randomly oriented fibers or filaments. The fibers may be of natural or man-made origin. Natural fibers may be selected from the group consisting of wheat straw fibers, rice straw fibers, flax fibers, bamboo fibers, wood pulp fibers, cotton fibers, jute fibers, hemp fibers, sisal fibers, bagasse fibers, Hesper aloe fibers, miscanthus, marine or fresh water algae/seaweeds, silk fibers, wool fibers, and combinations thereof. Another group of fibers may also be regenerated cellulose fibers, such as viscose, Lyocell (Tencel^{®}), rayon, modal, cellulose acetate fibers, acrylic fibers, cuprammonium rayon, regenerated protein fibers etc. Preferably, the natural fibers or modified natural fibers are selected from the group consisting of cellulose fibers (also referred to as pulp or airfelt) or modified cellulose fibers, such as intra-fiber crosslinked cellulose fibers, cotton fibers, bamboo fibers, viscose fibers or mixtures thereof. More preferably, the natural fibers or modified natural fibers are cellulose fibers or modified cellulose fibers. Synthetic fibers may be selected from the group consisting of polyolefins (such as polyethylene, polypropylene or combinations and mixtures thereof), polyethylene terephthalate (PET), co-PET, polylactic acid (PLA), polybutylene succinate (PBS), polyhydroxy alkanoid (PHA), nylon (or polyamide), or mixtures or combinations thereof.

The fibers in a nonwoven web are consolidated by friction, and/or entanglement, and/or cohesion, and/or adhesion, and/or by heat bonding, pressure bonding, or heat and pressure bonding, and/or ultrasonic bond, excluding paper and products which are woven, knitted, tufted, stitch-bonded. The fibers may be staple fibers (e.g. in carded nonwoven webs) or continuous fibers (e.g. in spunbonded or meltblown nonwoven webs).

The nonwoven webs comprised by the absorbent core of the present invention may comprise or may consist of superabsorbent fibers.

Nonwoven webs can be formed by many processes such as meltblowing, spunlaying, solvent spinning, electrospinning, and carding, and the fibers can be consolidated, e.g. by hydroentanglement (in spunlace nonwoven webs), air-through bonding (using hot air that is blown through the fiber layer in the thickness direction), infrared heat, needle-punching, one or more patterns of bonds and bond impressions created through localized compression and/or application of heat or ultrasonic energy, or a combination thereof. The fibers may, alternatively or in addition, be consolidated by use of a binder. The binder may be provided in the form of binder fibers or particles (which are subsequently molten) or may be provided in liquid, such as a styrene butadiene binder. A liquid binder is provided to the fibers (e.g. by spraying, printing or foam application) and is subsequently cured to solidify.

The basis weight of nonwoven webs is usually expressed in grams per square meter (g/m²). The basis weight a nonwoven web may be essentially constant throughout the nonwoven web or may be profiled.

The nonwoven web, especially nonwoven webs consisting of or comprising superabsorbent fibers, may be carded webs formed by needle-punching. In needle punching, the fibers cohesion and the interlacing of the fibers with one another is obtained by means of needles passing through a moving fibrous layer and causing the fibers to intermingle with one another. One or more, or all of the nonwoven webs of the absorbent core of the present invention may also be formed of two or more precursor webs, which are combined with each other by a needle punching process.

Alternatively, one or more, or all of the nonwoven webs of the absorbent core of the present invention may be formed by spunlacing. In a spunlace nonwoven web the fibers have been carded as precursor web and then subjected to hydroentanglement to intermingle and intertwine the fibers with each other. Cohesion and the interlacing of the fibers with one another may be obtained by means of a plurality of jets of water under pressure passing through a moving fleece or cloth and, like needles, causing the fibers to intermingle with one another (hereinafter also referred to as "hydraulic interlacing"). Thus, consolidation of a spunlace nonwoven web is essentially a result of hydraulic interlacing. "Spunlace nonwoven web", as used herein, also relates to a nonwoven web formed of two or more precursor webs, which are combined with each other by hydraulic interlacing.

The two or more webs, prior to being combined into one nonwoven by needle-punching or hydraulic interlacing, may have undergone bonding processes, such as heat and/or pressure bonding by using e.g. a patterned calendar roll and an anvil roll to impart a bonding pattern. However, the two or more webs are combined with each other solely by needle-punching or hydraulic interlacing.

Alternatively, the carded nonwoven web made by needle-punching or spunlacing is a single nonwoven web, i.e., it is not formed of two or more precursor webs. Still in another alternative, one or more, or all of the nonwoven webs of the absorbent core of the present invention may be formed of one precursor web onto which staple fibers are laid down. The staple fibers may be superabsorbent fibers or may comprise superabsorbent fibers. The staple fibers may not have been consolidated into a self-sustaining precursor web but the fibers are loosely laid onto the precursor web. The relatively loose staple fibers are then integrated and intertwined with each other and with the fibers of the underlying precursor web by (only) needle-punching or (only) hydraulic interlacing. Spunlace and/or needle punched nonwoven layers/webs can be made of staple fibers or continuous fibers (continuous fibers are also often referred to as filaments).

Through-air bonding (interchangeably used with the term "air-through bonding") means a process of bonding staple fibers or continuous fibers by forcing air through the nonwoven web, wherein the air is sufficiently hot to melt (or at least partly melt, or melt to a state where the fiber surface becomes sufficiently tacky) the polymer of a fiber or, if the fibers are multicomponent fibers, wherein the air is sufficiently hot to melt (or at least partly melt, or melt to a state where the fiber surface becomes sufficiently tacky) one of the polymers of which the fibers of the nonwoven web are made. The melting and re-solidification of the polymer provide the bonding between different fibers.

"Comprise," "comprising," and "comprises" are open ended terms, each specifies the presence of the feature that follows, e.g., a component, but does not preclude the presence of other features, e.g., elements, steps, components known in the art or disclosed herein. These terms based on the verb "comprise" encompasses the narrower terms "consisting essentially of" which excludes any element, step or ingredient not mentioned which materially affect the way the feature performs its function, and the term "consisting of' which excludes any element, step, or ingredient not specified.

"Body-facing" (also referred to as "skin-facing" herein) and "garment-facing" refer respectively to the relative location of an element or a surface of an element, layer, component. "Body-facing" implies the element or surface is nearer to the wearer during wear than another element of the same component. An example is the absorbent core having a body-facing surface, which is the surface of the absorbent core that is nearer to the body of the wearer than the opposite surface of the absorbent core, which is garment-facing. "Garment-facing" implies the element or a surface of an element, layer, component, is more remote from the wearer during wear than another element or a surface of an element, layer, component, or of the absorbent article as a whole. The garment-facing surface may face another (i.e. other than the wearable article) garment of the wearer, other items, such as the bedding, or the atmosphere. Typically, the body-facing surface of the topsheet forms a least a portion of the body-facing surface of the absorbent article as a whole, and the garment-facing surface of the outer cover nonwoven forms at least a portion of the garment-facing surface of the absorbent article as a whole.

### Folded Absorbent Articles

Absorbent articles are typically folded prior to being compressed and packaged. Diapers are typically folded once along the central transverse axis as fold line into a so-called bi-folded configuration. After folding, the body-facing surface is in a face-to-face configuration and the garment-facing components are facing outside. Further, for feminine hygiene articles, a so-called tri-folded configuration is common, in which the article is folded twice, once along a fold line which is located at around 1/3 of the overall longitudinal length, i.e. the length along the central longitudinal axis 50, of the article and then once along a fold line which is located at around 2/3 of the overall length of the article. Taped diapers and pants are more non-uniform in their cross-section than pads. In the bi-folded configuration, this non-uniformity is even more pronounced, as typically high caliper, high basis weight respectively, areas are folded onto each other, while low caliper, low basis weight respectively, areas are folded onto each other.

It has been discovered that by applying a combination of two folds to an absorbent article, packages with improved stability and stackability, which is reflected by the uniform and/ or low response to pressure reflected by the Package Out-of-Stack Waist Compression Deflection, the Package Out-of-Stack Compression Deflection Differential, the Package Out-of-Stack Waist Compression Deflection Factor, the Package Out-of-Stack Compression Deflection Factor Differential, the Package In-Stack Waist Compression Deflection Factor, and the Package In-Stack Compression Deflection Factor Differential discussed herein. The shifted center of gravity of the packages further contributes to increased stability. In addition, bag dimensions can be reduced in comparison to bi-folded diapers without applying to high compression pressure and thus potentially damaging the absorbent articles. This advantage can be further increase when the packages are provided with a certain in-bag stack height.

Both folds are substantially perpendicular to the central longitudinal axis 50, thus substantially parallel to the central lateral axis 48, and located in between the front waist edge of the absorbent core 112 and the back waist edge of the absorbent core 116. The first fold 400 extends transversely across the crotch region 14 along a first fold line substantially perpendicular to the central longitudinal axis 50. The second fold 500 extends transversely across the front and back waist regions 12, 16 along a second fold line. The location of the respective fold lines within the crotch region 14, front waist region 12 and back waist region 16 may vary depending on the type of absorbent article, the presence of certain components and materials etc. The location of the second fold line further may vary due to the location of the first fold line.

Due to the first fold 400, parts of the absorbent article 10 having a relative high caliper, relative high basis weight, and parts of the absorbent article having a relative low caliper, relative low basis weight, are folded onto another, overlapped respectively. By applying the second fold 500, parts of the bi-folded absorbent article with a relative low caliper, relative low basis weight respectively, are folded back onto and thus overlap with parts with a relative high caliper, relative high basis weight respectively. Due to such two folds, the folded articles exhibit uniformly distributed thickness and pressure response both in the out-of-plane-direction, which corresponds to the in-stack direction, i.e. the direction of the stack height, for stacked articles, as well as in the out-of-plane-direction, which corresponds to the out-of-stack direction for stacked articles, thus at the so-called fold noses.

The first fold line may divide the crotch region 14 into two parts of unequal length in the direction of the central longitudinal axis 50. Accordingly, the front end edge 18 and the back end edge 20 of the absorbent article may not be in contact in the bi-folded diaper. The central crotch area, i.e. the crotch region close to the central transverse axis 48, typically exhibits the highest caliper, highest basis weight respectively. By applying such an asymmetric first fold 400, the highest caliper areas are not folded onto themselves and thus the overall maximum caliper, the maximum basis weight, of the bi-folded article is reduced. This in turn, may facilitate matching this maximum caliper via the second fold 500 to obtain a folded article with a uniform caliper/ basis weight distribution.

In particular, the first fold line may be located closer to the front end edge 18 than to the back end edge 20 of the absorbent article 10. In other words, the first fold line may be located between the front end edge 18 and the central lateral axis 48. The first fold line may be located between front end edge 18 and the central lateral axis 48 and may have distance from the central lateral axis 48 in the direction of the longitudinal axis 50 of from 1.0 mm to 100.0 mm, from 5.0 mm to 75.0 mm, from 10.0 mm to 50.0 mm, from 15.0 mm to 45.0 mm or from 20.0 mm to 40.0 mm. In addition or alternatively, the front end edge 18 may have a distance from the back end edge 20 in the direction of the longitudinal axis 50 towards the first fold 400 of from 1.0 mm to 100.0 mm, from 5.0 mm to 75.0 mm, from 10.0 mm to 50.0 mm, from 15.0 mm to 45.0 mm or from 20.0 mm to 40.0 mm in the bi-folded article 10. Due to such a shifted first fold line and thus asymmetric first fold 400, the wearer-facing surface of the back waist region 14 near the back end edge 20 is exposed in the bi-folded article creating a low caliper, low basis weight, area, which can be folded onto the relatively larger caliper, larger basis weight, parts of the bi-folded crotch region via the second fold 500 to obtain uniformly distributed thickness and pressure response in an article comprising two folds.

Such an asymmetric first fold 400 may exemplarily be applied to absorbent articles 10 in the form of a taped diaper. Taped diapers contain fasteners 46 and back ears 42 in the back waist region 16 and front ears 47 in the front waist region 12. These components are typically folded towards the central longitudinal axis 50 prior to the first fold and may vary in caliper and basis weight, thus requiring such an asymmetric fold to obtain uniformly distributed thickness and pressure response in an article comprising two folds.

In one example, the diapers and/ or pants are in open form. In the open form, side portions may be folded over and laterally inward about longitudinal side edge fold lines more freely than in closed form. In particular an open form may be used for articles in which the first fold line may be located closer to the front end edge 18 than to the back end edge 20 of the absorbent article 10. In the open form the front and back end edges 18, 20 can be moved for freely with respect to each other than in closed form. Generally the folding operation can be integrated more easily in existing manufacturing set-ups for absorbent articles in open form.

The first fold line may substantially correspond to the central lateral axis 48. Thus, the first fold line may substantially bisect the crotch region 14 into two halves of substantially equal length in the direction of the central longitudinal axis 50. Accordingly, the front end edge 18 and the back end edge 20 of the absorbent article 10 may be adjacent in the bi-folded diaper. Such a first fold 400 may be applied to absorbent articles 10 is in the form of a pant. In pant-type articles the front waist region 12 and back waist region 16 are more similar than for taped diapers. Due the front 54 and back belt 56 materials pant-type articles have a higher caliper, basis weight, near the front end and back end edges 18, 20 than taped-diapers. Thus, the large caliper created by folding the central crotch region, typically the area with the highest caliper, onto itself via the first fold 400, can be substantially matched by having the front 54 and back belt 56 folded onto another in the first folding operation and then folded onto parts of the bi-folded diaper with higher caliper than the combined front 54 and back belt 56 but lower caliper than the combined central crotch region via the second fold 500.

The second fold 500 extends transversely across the front and back waist regions 12, 16 along a second fold line, which is substantially perpendicular to the central longitudinal axis 50. The second fold 500 occurs after the first fold 400 and thus in the bi-folded article. The distance of the second fold line to the front end edge 18 may be different than the distance to the back end edge 20 in the bi-folded configuration. In particular, the second fold line may be located closer to the front end edge 18 than to the back end edge 20. The second fold line may be located from 1.0 mm to 100.0 mm, from 5.0 mm to 75.0 mm, from 10.0 mm to 50.0 mm, from 15.0 mm to 45.0 mm or from 20.0 mm to 40.0 mm closer to the front end edge 18 than to the back end edge 20. Due second fold line being closer to the front end edge 18, a low caliper, low basis weight, area of the bi-folded article, where only the back waist region 16 is present, is folded onto the relatively larger caliper, larger basis weight, parts of the bi-folded crotch region to obtain uniformly distributed thickness and pressure response in an article comprising two folds.

The second fold line may lie between the first fold line and the front waist edge of the absorbent core 112 in the front waist region 12 and/ or may lie between the first fold line and the back waist edge of the absorbent core 116 in the back waist region 16. In other words, the second fold line may lie within the core but not correspond to the core edges 112, 116. Typically, absorbent articles have the highest caliper, basis weight respectively, in the crotch region 14, in particular in the central crotch region close to the central transverse axis 48. Typically, the basis weight decreases from the central transverse axis 48 towards the front end edge 18 and from the central transverse axis 48 towards the back end edge 20. This is in particular pronounced for absorbent articles with bulky absorbent materials 72 in the absorbent core 30 or comprising an acquisition and distribution system 38.

The absorbent article 10 may further comprise an acquisition and distribution system 38 at least partially positioned intermediate the absorbent core 30 and the topsheet 26. The crotch region 14 may at least partially comprise the acquisition and distribution system 38. The absorbent core 30 may extend beyond the acquisition and distribution system 38 in both the front waist region 12 and the back waist region 16. In other words, the front waist edge of the absorbent core 112 may be closer to the front end edge 18 than the front waist edge 212 of acquisition and distribution system 38 and the back waist edge of the absorbent core 116 is closer to the back end edge 20 than the back waist edge 216 of acquisition and distribution system 38. The acquisition and distribution system 38 may have a non-uniformly distributed basis weight at least in the direction of the longitudinal axis 50. The acquisition and distribution system 38 may have its maximum basis weight in the crotch region 14, in particular at or close to the central transverse axis of the acquisition and distribution system 248, and the basis weight may decrease in the direction of the longitudinal axis 50 towards the front waist edge of the acquisition and distribution system 212 and/ or the back waist edge of the acquisition and distribution system 216. Such decrease in basis weight of acquisition and distribution system 38 may be gradually, steadily, linearly or stepwise. By such basis weight distribution, relatively low basis weight parts of the acquisition and distribution system 38 are folded onto relatively high basis weight parts by the second fold 500 increasing the uniform basis weight distribution and thus pressure response of the folded absorbent article 10.

For absorbent articles comprising an acquisition and distribution system 38, the second fold line may either correspond to a front waist edge of the acquisition and distribution system 212 or may lie between the front waist edge of the acquisition and distribution system 212 and the front waist edge of the absorbent core 112 in the front waist region 12. For clarification, in such an example, the second fold line does not correspond to the and the front waist edge of the absorbent core 112. Further, the second fold line may either correspond to a back waist edge of the acquisition and distribution system 216 or lie between the back waist edge of the acquisition and distribution system 216 and the back waist edge of the absorbent core 116 in the back waist region 16. The first fold line may substantially correspond to the central transverse axis of the acquisition and distribution system 248.

In one example, the absorbent cores 30 may comprise absorbent material 72 comprising a blend of cellulose fibers and superabsorbent particles; and/ or high internal phase emulsion foam. The absorbent material 72 may be non-uniformly distributed at least in the direction of the longitudinal axis. The absorbent material 72 may have its maximum basis weight in the crotch region 14, in particular at or close to the central transverse axis 48, and the basis weight may decrease in the direction of the longitudinal axis 50 towards the front waist edge of the absorbent core 112 and/ or the back waist edge of the absorbent core 116. Such decrease in basis weight of absorbent material 72 may be gradually, steadily, linearly or stepwise. By such basis weight distribution, relatively low basis weight parts of the absorbent core 30 are folded onto relatively high basis weight parts by the second fold 500 increasing the uniform basis weight distribution and thus pressure response of the folded absorbent article 10.

In other embodiments, the absorbent core 30 may comprise less than 5% by weight of cellulose, more preferably less than 2% and most preferably is cellulose free. The resulting absorbent structures have a reduced thickness in the dry state compared to conventional absorbent structure comprising cellulosic fibers. The reduced thickness helps to improve the fit and comfort of the absorbent article for the wearer. In addition, a thinner core 30 may reduce the overall maximum caliper, the maximum basis weight, of the bi-folded article. This in turn, may facilitate matching this maximum caliper via the second fold 500 to obtain an article comprising two folds with a uniform caliper/ basis weight distribution.

The backsheet 28 may comprise a breathable film. Hence, the backsheet 28 may be breathable, thus vapor pervious, but liquid impervious. The backsheet may prevent the fluids absorbed and contained in the absorbent structure from wetting materials that contact the absorbent article such as underpants, pants, pyjamas, undergarments, and shirts or jackets, thereby acting as a barrier to fluid transport. The backsheet may also allow the transfer of at least water vapor, or both water vapor and air through it. The backsheet may be elastic or inelastic. Such breathable films may be less stiff and flexible than common backsheet films. Hence, they may be more prone to SAP particles potentially creating so-called pinholes during compression of the absorbent articles and thus compromising the backsheet barrier function. Such risk of failure can be reduced by the more uniformly distributed pressure response within the article of the present disclosure. In particular, an in-bag stack height of from 70 mm to 110 mm with the folded absorbent articles having breathable backsheet 28 greatly reduces the likelihood of pinhole creation.

The absorbent article 10 may further comprise an elastic waistband 36. For absorbent articles having a non-uniform pressure response - in contrast to the articles of the present disclosure, such elastic waistbands may experience bunching and more generally strains. This may result in in compromised elasticity and make the material selection more challenging to ensure the recovery potential of the elastic waistband 36. In addition to the bunching during compression, such bunching may occur, when pressure is applied from the waist out-of-stack side, e.g. when packages of absorbent articles are stacked on a pallet. In one example, the absorbent article 10 may comprise an elastic waistband 36 in the back waist region 16. The first fold line may be located such that the elastic waistband 36 in the back waist region 16 is not overlapped by the front waist region 12 in the bi-folded absorbent article 10. In other words, in the bi-folded configuration, the front end edge 18 may be closer to the first fold nose than the lower edge, i.e. the edge closer to the central transverse axis 48, of the elastic waistband 36 of the back waist region 16. As already discussed, the back waist region 16 in proximity to the back waist edge 20 typically has a higher caliper, basis weight respectively, than the front waist region 12 in proximity to the front end edge, in particular for taped-diapers. The elastic waistband 36 in the back waist region 16 further increases this difference. Hence, having parts of the back waist region 16 close to the back waist edge 20 exposed, facilitates obtaining an article comprising two folds with a uniform caliper/ basis weight distribution and pressure response.

The absorbent article 10 may comprise one or more channels 76, wherein the second fold line lies between the front waist edge of the one or more channels 76 and the front waist edge of the absorbent core 112 and/ or between the back waist edge of the one or more channels 76 and the back waist edge of the absorbent core 116 in the back waist region 16. The front waist edge of the one or more channels 76 corresponds to the line, which is perpendicular to the central longitudinal axis 50 and drawn through the point of one or more channels 76 closest to the front end edge 18. The back waist edge of one or more channels 76 corresponds to the line, which is perpendicular to the central longitudinal axis 50 and drawn through the point of the one or more channels 76 closest to the back end edge 20.

The folding process may be carried out by any method known in the art. For example US 8,986,184, "Apparatuses and Methods for Folding an Absorbent Article", and EP3789328A1, "Method and Apparatus for Diverting and/or Folding Articles", both incorporated herein by reference, disclose folding processes.

### Packages comprising Folded Absorbent Article

Packages comprising folded articles with a uniform pressure response are provided. The pressure response is uniform when pressure is applied along different locations of one side of a package, as well as when pressure is applied on opposing sides of a package. By this, it is ensured that the packages, stacks of packages and pallets comprising a plurality of packages/ stacks of a plurality of packages are stable on shelf, in transport and during warehousing. Further, the packages of the present disclosure provide reduced packaging dimensions due to the higher absorbent article density within the package without exposing the encased articles to the rigors of higher compression forces.

In particular, the pressure response of the packages is reflected by the Compression Deflection at different pressures and the slope of such compression deflection with increasing pressure as detailed in the following. The response is measured for pressure applied in the stacking direction (In-Stack parameters); and for pressure applied from the direction of the respective fold noses of the first 400 and/ or second fold 400 (Out-of-Stack).

The package 1 may have a Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm according to the Package Compression Test detailed herein. The package 1 may have a Package Out-of-Stack Waist Compression Deflection at 0.6 psi of at least 0.0 mm or of at least 0.5 mm according to the Package Compression Test detailed herein. The Package Out-of-Stack Waist Compression Deflection reflects the pressure response, when pressure is applied at the fold nose of the second fold 500 from the direction of the tip of the fold nose of the folded absorbent articles and packages containing them. For a package with bi-folded article the pressure would be applied at the front waist edge 18 and/ or the back waist edge 20. Typically, packages would be arranged such that this would reflect the pressure response of the top panel of the package. The pressure of 0.6 psi was chosen to reflect the pressure experienced by a package forming part of the bottommost layer of packages in a standard pallet. The low Package Out-of-Stack Waist Compression Deflection values at 0.6 psi of the packages of the present disclosure show that a uniform surface without or with minimal deformation defects of a single package and/ or layers of similar packages can be achieved by employing the packages of the present disclosure. This holds true even if the experienced pressure may vary for different location of the package or different packages of the layer of packages. Thus, pallets with packages, layers of packages respectively, of the present disclosure exhibit increased stability.

The package 1 may have a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm according to the Package Compression Test detailed herein. The package 1 may have a Package Out-of-Stack Compression Deflection Differential at 0.6 psi of from -3.0 to 3.0 mm, preferably from -2.5 to 2.5 mm, more preferably from -2.0 to 2.0 mm, even more preferably from -1.5 to 1.5 mm, most preferably from -1.0 to 1.0 mm or even from -0.5 to 0.5 mm according to the Package Compression Test detailed herein. The Package Out-of-Stack Compression Deflection Differential at 0.6 psi reflects the difference in pressure response of the when pressure is applied at the fold nose of the second fold 500 vs. when pressure is applied at the fold nose of the first fold 400 from the direction of the respective tips of the fold nose of the folded absorbent articles and packages containing them. Thus, the low Package Out-of-Stack Waist Compression Deflection Differential values at 0.6 psi of the packages of the present disclosure show that a substantially uniform surface without or with minimal deformation defects of a single package and/ or layers of similar packages can be achieved by employing the packages of the present disclosure and that the pressure response of the top and bottom panel of the packages is uniform. Hence, the by having a low Package Out-of-Stack Waist Compression Deflection Differential at 0.6 psi pallet stability can be increased, as both top and bottom panel and thus also adjacent panels at the interface between two layers of packages show similar pressure responses.

The package 1 may have a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm according to the Package Compression Test detailed herein. The low Package Out-of-Stack Waist Compression Deflection values at 5.0 psi the pressure response, when pressure is applied at the fold nose of the second fold 500 from the direction of the tip of the fold nose of the folded absorbent articles and packages containing them. The pressure of 5.0 psi was chosen to reflect situation in which packages may experience high-pressure, for example if a plurality of pallets is stacked in warehousing. In a typical arrangement of packages on a pallet, the uppermost surface would correspond to the Out-of-Stack Waist side of the packages. Thus, the low Package Out-of-Stack Waist Compression Deflection values at 5.0 psi of the packages of the present disclosure lead to increased stability of stacks of pallets.

The package 1 may have a Package Out-of-Stack Compression Deflection Differential at 5.0 psi of at most 8.0 mm, preferably at most 7.0 mm, more preferably at most 6.0 mm, even more preferably at most 5.0 mm, most preferably at most 4.0 mm or even at most 3.0 mm according to the Package Compression Test detailed herein. The package 1 may have a Package Out-of-Stack Compression Deflection Differential at 5.0 psi of from -8.0 mm to 8.0 mm, preferably from -7.0 mm to 7.0 mm, more preferably from -6.0 mm to 6.0 mm, even more preferably from -5.0 mm to 5.0 mm, most preferably from -4.0 mm to 4.0 mm or even from -3.0 mm to 3.0 mm according to the Package Compression Test detailed herein. Analogously to previous discussions, the low Package Out-of-Stack Waist Compression Deflection Differential values at 5.0 psi of the packages of the present disclosure lead to increased stability of stacks of pallets.

The package 1 may have a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi according to the Package Compression Test detailed herein.

The package 1 may have a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi according to the Package Compression Test detailed herein. The package 1 may have a Package Out-of-Stack Compression Deflection Factor Differential of from -0.80 mm/ psi to 0.80 mm/ psi, preferably from -0.50 mm/ psi to 0.50 mm/ psi, more preferably from -0.40 mm/ psi to 0.40 mm/ psi, even more preferably from -0.30 mm/ psi to 0.30 mm/ psi, most preferably from -0.20 mm/ psi to 0.20 mm/ psi or even from -0.10 mm/ psi to 0.10 mm/ psi according to the Package Compression Test detailed herein.

The package 1 may have a Package In-Stack Waist Compression Deflection Factor of at most 4.50 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi according to the Package Compression Test detailed herein. The In-Stack Waist Compression Deflection Factor reflects the pressure response with increasing pressure, when pressure is applied at the fold nose of the second fold 500 from a direction perpendicular to the plane of the absorbent article, parallel to the direction of stack height respectively, of the folded absorbent articles and packages containing them. For a package with bi-folded article the pressure would be applied at the front waist edge 18 and/ or the back waist edge 20. Typically, packages would be arranged such that this would reflect the pressure response of the top or bottom part of a side panel of the package. The response is measured as slope and is relatively linear for the packages of the present disclosure. Thus, stability of stacks of packages can also be increased, if the stacking of packages occurs in a direction parallel to the stack height direction of the folded absorbent articles.

The package 1 may have a Package In-Stack Waist Compression Deflection at 0.6 psi of at most 2.75 mm, preferably at most 2.50 mm, more preferably at most 2.25 mm, even more preferably at most 2.00 mm, most preferably at most 1.75 mm or even at most 1.50 mm according to the Package Compression Test detailed herein. The Package In-Stack Waist Compression Deflection reflects the pressure response, when pressure is applied at the fold nose of the second fold 500 from a direction perpendicular to the plane of the absorbent article, parallel to the direction of stack height respectively, of the folded absorbent articles and packages containing them. For a package with bi-folded article the pressure would be applied at the front waist edge 18 and/ or the back waist edge 20. Typically, packages would be arranged such that this would reflect the pressure response of the top or bottom part of a side panel of the package. Thus, stability of stacks of packages can also be increased, if the stacking of packages occurs in a direction parallel to the stack height direction of the folded absorbent articles. Such stability may be in particular beneficial, when pallets are turned by 90° for stacking to optimally utilize the space of shipping containers.

The package 1 may have a Package In-Stack Compression Deflection Differential at 0.6 psi of at most 1.00 mm, preferably at most 0.80 mm, more preferably at most 0.60 mm, even more preferably at most 0.40 mm, most preferably at most 0.30 mm or even at most 0.20 mm according to the Package Compression Test detailed herein. The package 1 may have a Package In-Stack Compression Deflection Differential at 0.6 psi of from -1.50 to 1.00 mm, preferably from -1.00 to 0.80 mm, more preferably from -0.60 to 0.60 mm, even more preferably from -0.40 to 0.40 mm, most preferably from -0.30 to 0.30 mm or even from -0.20 to 0.20 mm according to the Package Compression Test detailed herein. The Package In-Stack Compression Deflection Differential at 0.6 psi reflects the difference in pressure response of the when pressure is applied at the fold nose of the second fold 500 vs. when pressure is applied at the fold nose of the first fold 400 from a direction perpendicular to the plane of the absorbent article, parallel to the direction of stack height respectively, of the folded absorbent articles and packages containing them. Thus, the low Package In-Stack Compression Deflection Differential values at 0.6 psi of the packages of the present disclosure show that a substantially uniform surface without or with minimal deformation defects of a single package and/ or layers of similar packages can be achieved by employing the packages of the present disclosure and that the pressure response along a side panel of the packages is uniform.

The package 1 may have an in-bag stack height of from 70 mm to 110 mm, according to the In-Bag Stack Height Test detailed herein. As already discussed before, by such an in in-bag stack height range of the packages, a compression level of the absorbent articles is achieved with an optimal balance between avoidance of damages to the backsheet 28 due to SAP pinholes are avoided on the one hand, while on the other hand an adequate amount of absorbent articles per package volume can be provided.

The package 1 may have Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein. Due to the center of gravity being located closer to the bottom of the package on-shelf stability and stability within a stack of packages is further increased.

Packages 1 of the present disclosure comprise a plurality of absorbent articles in the form of diapers and/ or pant as described in the previous section.

In particular, the package 1 comprises a plurality of absorbent articles 10 in the form of diapers and/ or pants, wherein each of the plurality of absorbent articles 10 comprises a liquid permeable topsheet 26, a liquid impermeable backsheet 28, and an absorbent core 30 at least partially positioned between the topsheet 26 and the backsheet 28. Each article further comprises a front waist region 12, a back waist region 16, a crotch region 14 located between the front waist region 12 and the back waist region 16 and a central longitudinal axis 50. The articles are folded twice. As such they comprise a first fold 400 extending transversely across the crotch region 14 along a first fold line, which is substantially perpendicular to the central longitudinal axis 50; and further comprise a second fold 500 extending transversely across the front and back waist regions 12, 16 along a second fold line, which is substantially perpendicular to the central longitudinal axis 50.

The plurality of folded articles 10 may be placed in similar orientation within a package of the present disclosure. The plurality of absorbent articles 10 may be arranged in a single horizontal row, a single stack respectively, or in one or more vertical stacks. The articles 10 may be arranged in one or more vertical stacks and all the articles 10 of one stack may be oriented in the same direction. In other words, the fold nose of the first fold 400 of each article 10 is aligned in the same direction, while the fold nose of the second fold 500 is aligned in the opposing direction. When two or more stacks are present in the package 1, the articles of the respective two or more stacks may be oriented in the same direction, Thus the fold noses of the first fold 400 of the articles of a first stack make up one side of the package 1, while the fold noses of the second fold 500 of the articles of a different stack make up the opposing side of the package 1. In another example, the plurality of folded articles 10 comprised by a first stack may have the fold noses of the first fold 400 oriented along one side of the stack, and the plurality of folded articles 10 of the neighboring stack may be rotated 180 degrees. Thus, the respective fold noses of the first fold 400 of one stack are in contact with the fold noses of the first fold 400 of the neighboring stack or the respective fold noses of the second fold 500 of one stack are in contact with the fold noses of the second fold 500 of the neighboring stack. Due to the uniform pressure response from the side of the first fold 400 and the second fold 500, packages with two or more stacks - analogously to single stack packages - exhibit better stability and standability on shelf, during transport or in warehousing. In particular, the interface between vertically adjacent stacks is more uniform in packages of the present disclosure in comparison to e.g. packages with stacks of bi-folded absorbent articles.

The package 1 comprises a flexible package material. The flexible package material may comprise, essentially consist of or consist of one or more materials selected from the group consisting of polymeric film, paper, paper based material, paper with one or more barrier layers, and a paper/film laminate. The basis weight of the package material may be in the range of about 50 gsm to about 100 gsm or about 60 gsm to about 90 gsm. This basis weight ensures that the package material is flexible without feeling flimsy and is strong enough to withstand the rigors of packaging operations and/ or transport.

The packages may comprise polymeric films comprising recycled material, such as about 20% to about 100%, about 30% to about 90%, about 30% to about 80%, about 40% to about 60%, or about 50% recycled material. The recycled material may comprise post-industrial recycled material (PIR) and/or post-consumer recycled material (PCR). In some instances, the polymeric films used for the packages may comprise two outer layers and one or more inner layers. The one or more inner layers may comprise the recycled material or may comprise more recycled material than the outer layers. The recycled material may comprise recycled polyethylene. The recycled material may comprise recycled polyethylene PIR from trim from the packaging operation. By employing recycled material to the packages with folded absorbent articles, the environmental footprint of the packages of absorbent articles may further reduced.

The package material may comprise paper, paper based material, paper with one or more barrier layers, or a paper/film laminate. By employing such material, the environmental footprint of the packages of absorbent articles may further reduced. The package material may be in the range of about 50 gsm to about 100 gsm or about 60 gsm to about 90 gsm. This basis weight ensures that the package material is flexible without feeling flimsy and is strong enough to withstand the rigors of packaging operations and/ or transport. The one or more barrier layers may be in the range of about 3 gsm to about 15 gsm. The paper based package material with or without one or more barrier layers may exhibit a machine direction tensile strength of at least 5.0 kN/m, a machine direction stretch of at least 3 percent, a cross-machine direction tensile strength of at least 3 kN/m, and a cross-direction stretch at break of at least 4 percent, each as determined via ISO 1924-3. The paper based package material or paper based package material comprising a barrier layer or film may be recyclable or recyclable in normal paper recycling operations. The recyclability extent of the paper based package may be determined via recyclable percentage. The paper based package of the present disclosure may exhibit recyclable percentages of 70 percent or greater, 80 percent or greater, or 90 percent or greater. The paper based package of the present disclosure may have a recyclable percentage of between 70 percent to about 99.9 percent, between about 80 percent to about 99.9 percent, or between about 90 percent to about 99.9 percent. In one example, the package material of the present disclosure may exhibit a recyclable percentage of from about 95 percent to about 99.9 percent, from about 97 percent to about 99.9 percent, or from about 98 percent to about 99.9 percent. The recyclable percentage of the paper based package may be determined via test PTS-RH:021/97 (Draft Oct. 2019) under category II, as performed by Papiertechnische Stiftung located at Pirnaer Strasse 37, 01809 Heidenau, Germany. In another instance, the paper based packages of the present disclosure may exhibit an overall "pass" test outcome as determined by PTS-RH:021/97 (Draft Oct. 2019) under category II method. Any of the paper based packages may have opening features, such as lines of perforation, and may also have handles.

Graphics and/or indicia relating to properties of the absorbent articles may be formed on, printed on, positioned on, and/or placed on outer portions of the packages.

Each of the plurality of absorbent articles 10 in the package may be compressed. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

### General Description of an Absorbent Article

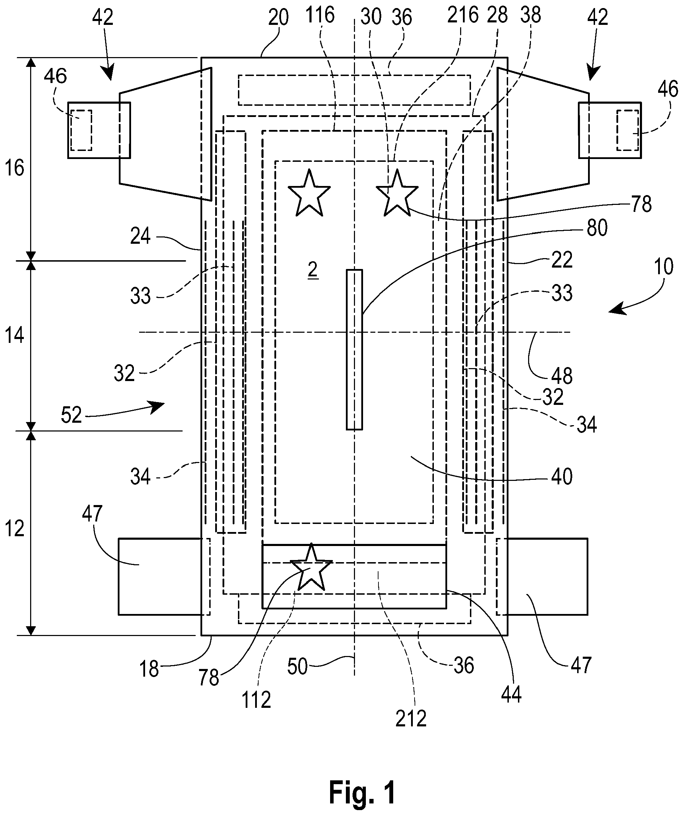

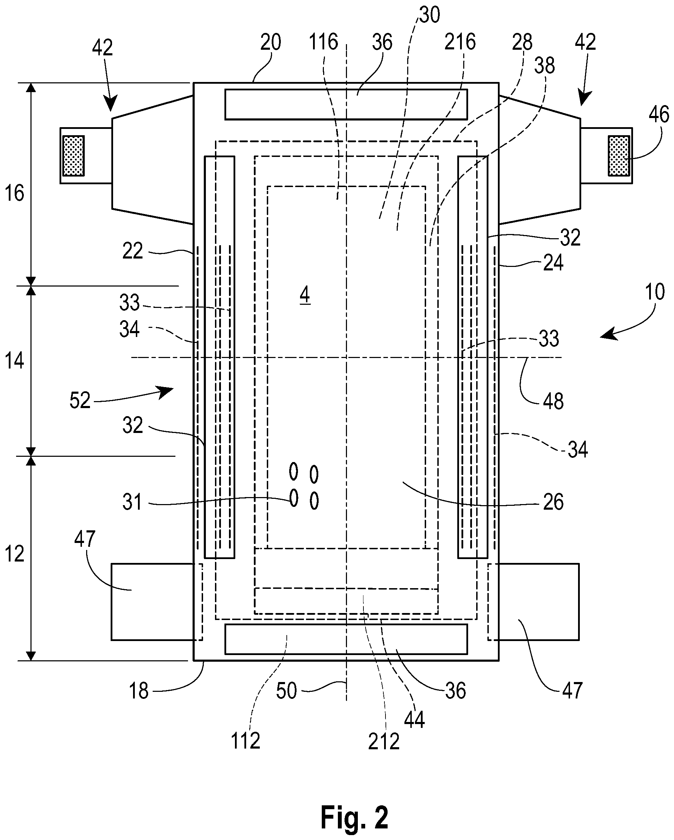

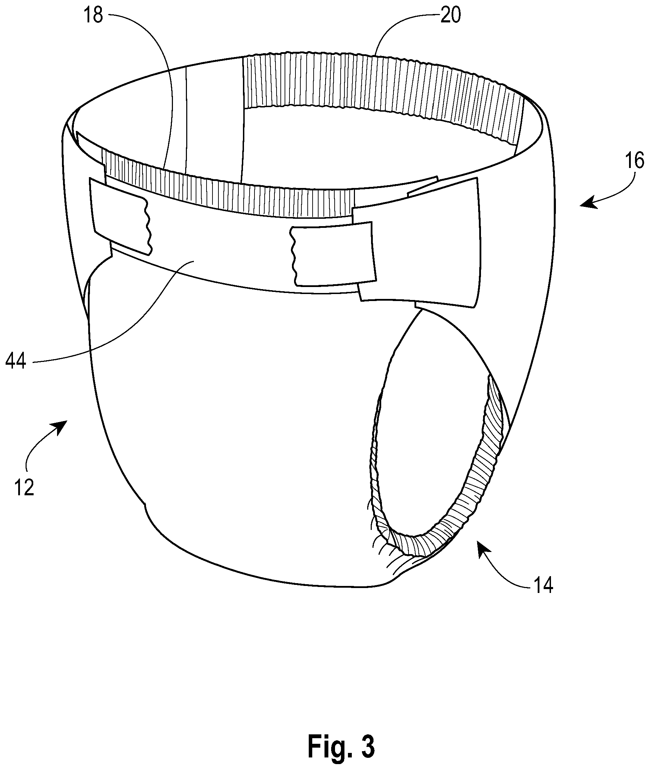

An example absorbent article 10 according to the present disclosure, shown in the form of a taped diaper, is represented in Figs. 1-3. Fig. 1 is a plan view of the example absorbent article 10, garment-facing surface 2 facing the viewer in a flat, laid-out state (i.e., no elastic contraction). Fig. 2 is a plan view of the example absorbent article 10 of Fig. 1, wearer-facing surface 4 facing the viewer in a flat, laid-out state. Fig. 3 is a front perspective view of the absorbent article 10 of Figs. 1 and 2 in a fastened configuration. The absorbent article 10 of Figs. 1-3 is shown for illustration purposes only as the present disclosure may be used for making a wide variety of diapers, including adult incontinence products, pants, or other absorbent articles, such as sanitary napkins and absorbent pads, for example.

The absorbent article 10 comprises a front waist region 12, a crotch region 14, and a back waist region 16. The crotch region 14 extends intermediate the front waist region 12 and the back waist region 16. The front waist region 12, the crotch region 14, and the back waist region 16 are each 1/3 of the length of the absorbent article 10. The absorbent article 10 comprises a front end edge 18, a back end edge 20 opposite to the front end edge 18, and longitudinally extending, transversely opposed side edges 22 and 24 defined by the chassis 52.

The absorbent core comprises front a waist edge 112 in the front waist region 12 and back waist edge 116 in the back waist region 16. The front waist edge of the absorbent core 112 corresponds to the line, which is perpendicular to the central longitudinal axis 50 and drawn through the point of the absorbent core 30 closest to the front end edge 18. The back waist edge of the absorbent core 116 corresponds to the line, which is perpendicular to the central longitudinal axis 50 and drawn through the point of the absorbent core 30 closest to the back end edge 20.

The absorbent article 10 comprises a liquid permeable topsheet 26, a liquid impermeable backsheet 28, and an absorbent core 30 positioned at least partially intermediate the topsheet 26 and the backsheet 28. The absorbent article 10 may also comprise one or more pairs of barrier leg cuffs 32 with or without elastics 33, one or more pairs of leg elastics 34, one or more elastic waistbands 36, and/or acquisition and distribution system 38. The acquisition and distribution system 38 may be positioned intermediate the topsheet 26 and the absorbent core 30. An outer cover material 40, such as a nonwoven material, may cover a garment-facing side of the backsheet 28. The absorbent article 10 may comprise back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 may also have front ears 47 in the front waist region 12. The absorbent article 10 has a central lateral (or transverse) axis 48 and a central longitudinal axis 50. The central lateral axis 48 extends perpendicular to the central longitudinal axis 50.

In other instances, the absorbent article may be in the form of a pant having permanent or refastenable side seams. Suitable refastenable seams are disclosed in U.S. Pat. Appl. Pub. No. 2014/0005020 and U.S. Pat. No. 9,421,137.

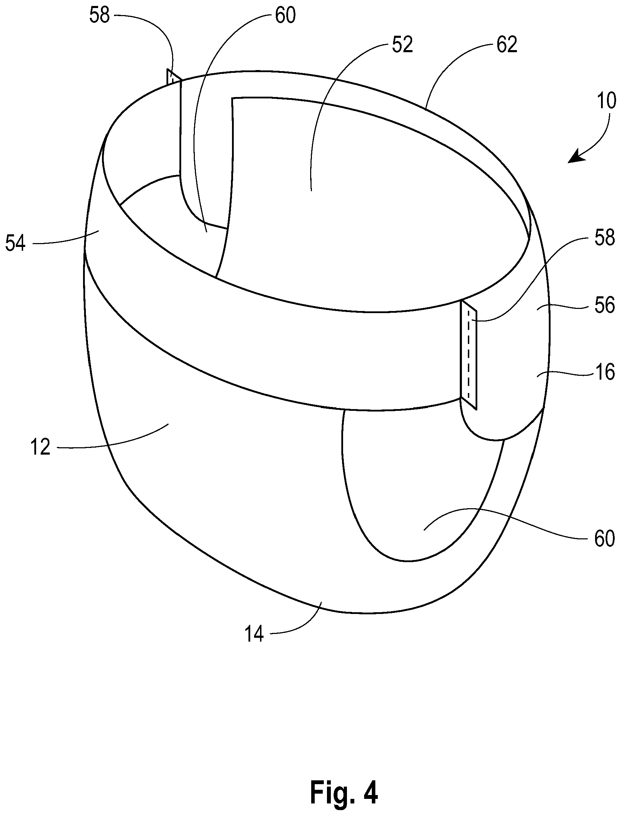

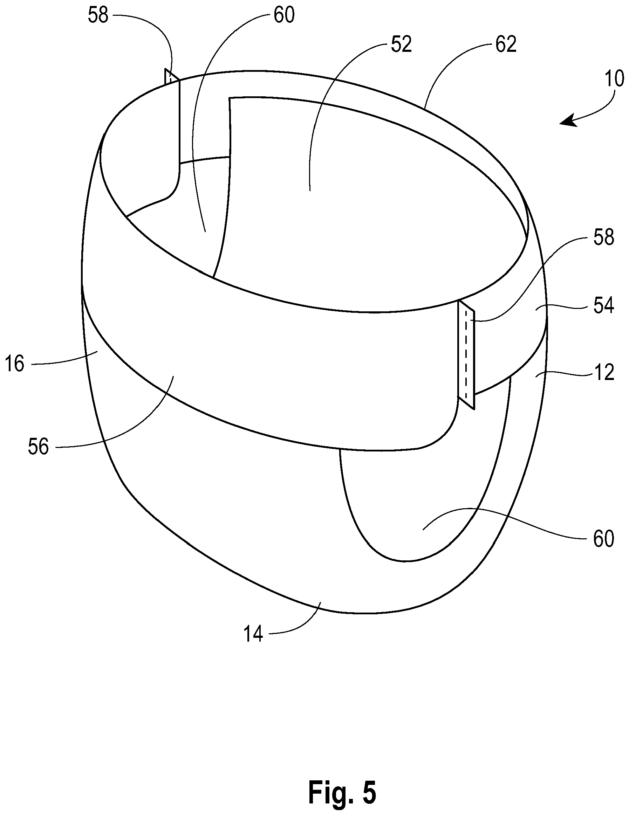

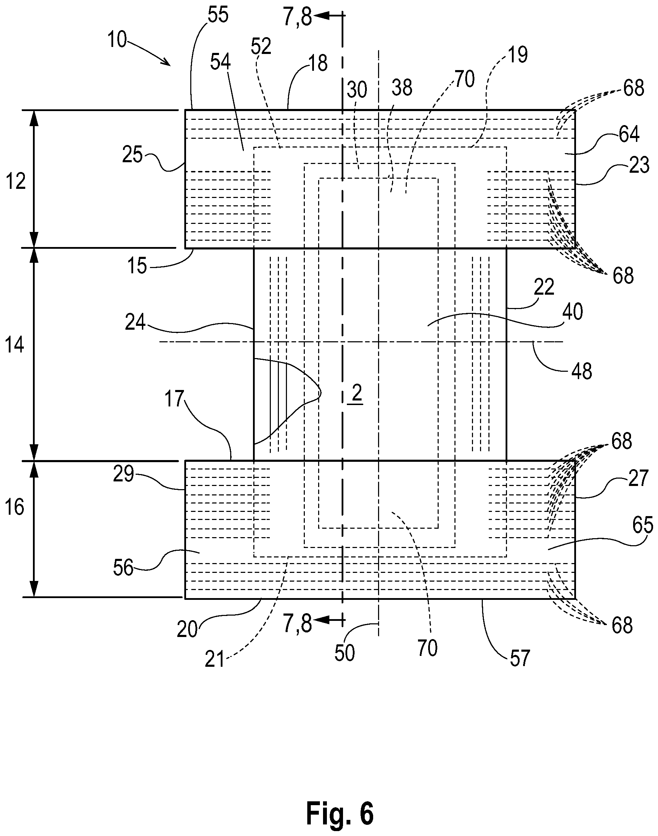

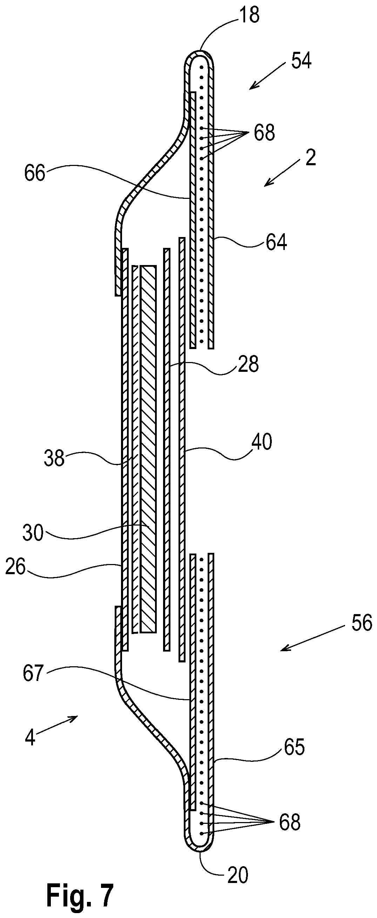

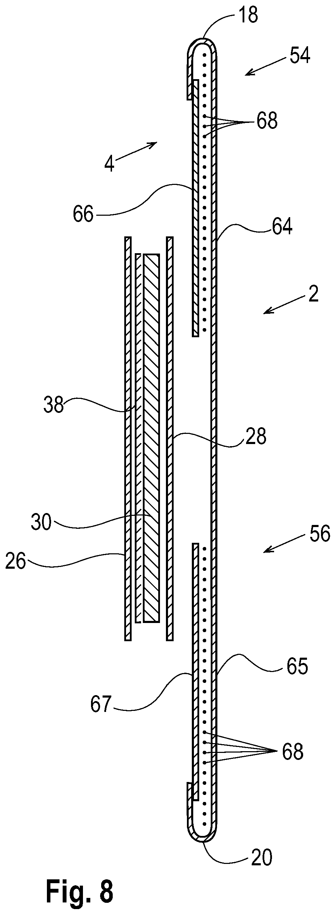

Referring to Figs. 4-8, an example absorbent article 10 in the form of a pant is illustrated. Fig. 4 is a front perspective view of the absorbent article 10. Fig. 5 is a rear perspective view of the absorbent article 10. Fig. 6 is a plan view of the absorbent article 10, laid flat, with the garment-facing surface facing the viewer. Elements of Fig. 4-8 having the same reference number as described above with respect to Figs. 1-3 may be the same element (e.g., absorbent core 30). Fig. 7 is an example cross-sectional view of the absorbent article taken about line 7-7 of Fig. 6. Fig. 8 is an example cross-sectional view of the absorbent article taken about line 8-8 of Fig. 6. Figs. 7 and 8 illustrate example forms of front and back belts 54, 56. The absorbent article 10 may have a front waist region 12, a crotch region 14, and a back waist region 16. Each of the regions 12, 14, and 16 may be 1/3 of the length of the absorbent article 10. The absorbent article 10 may have a chassis 52 (sometimes referred to as a central chassis or central panel) comprising a topsheet 26, a backsheet 28, and an absorbent core 30 disposed at least partially intermediate the topsheet 26 and the backsheet 28, and an optional acquisition and sitribution system 38, similar to that as described above with respect to Figs. 1-3. The absorbent article 10 may comprise a front belt 54 in the front waist region 12 and a back belt 56 in the back waist region 16. The chassis 52 may be joined to a wearer-facing surface 4 of the front and back belts 54, 56 or to a garment-facing surface 2 of the belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the absorbent article 10 in the form of a pant has two leg openings 60 and a waist opening circumference 62. The side seams 58 may be permanently joined using adhesives or bonds, for example, or may be refastenably closed using hook and loop fasteners, for example.

### Belts

Referring to Figs. 7 and 8, the front and back belts 54 and 56 may comprise front and back inner belt layers 66 and 67 and front and back outer belt layers 64 and 65 having an elastomeric material (e.g., strands 68 or a film (which may be apertured)) disposed at least partially therebetween. The elastic elements 68 or the film may be relaxed (including being cut) to reduce elastic strain over the absorbent core 30 or, may alternatively, run continuously across the absorbent core 30. The elastics elements 68 may have uniform or variable spacing therebetween in any portion of the belts. The elastic elements 68 may also be pre-strained the same amount or different amounts. The front and/or back belts 54 and 56 may have one or more elastic element free zones 70 where the chassis 52 overlaps the belts 54, 56. In other instances, at least some of the elastic elements 68 may extend continuously across the chassis 52.

The front and back inner belt layers 66, 67 and the front and back outer belt layers 64, 65 may be joined using adhesives, heat bonds, pressure bonds or thermoplastic bonds. Various suitable belt layer configurations can be found in U.S. Pat. No. 9,072,632.

Front and back belt end edges 55 and 57 may extend longitudinally beyond the front and back chassis end edges 19 and 21 (as shown in Fig. 6) or they may be co-terminus. The front and back belt side edges 23, 25, 27, and 29 may extend laterally beyond the chassis side edges 22 and 24. The front and back belts 54 and 56 may be continuous (i.e., having at least one layer that is continuous) from belt side edge to belt side edge (e.g., the transverse distances from 23 to 25 and from 27 to 29). Alternatively, the front and back belts 54 and 56 may be discontinuous from belt side edge to belt side edge (e.g., the transverse distances from 23 to 25 and 27 to 29), such that they are discrete.

As disclosed in U.S. Pat. No. 7,901,393, the longitudinal length (along the central longitudinal axis 50) of the back belt 56 may be greater than the longitudinal length of the front belt 54, and this may be particularly useful for increased buttocks coverage when the back belt 56 has a greater longitudinal length versus the front belt 54 adjacent to or immediately adjacent to the side seams 58.

The front outer belt layer 64 and the back outer belt layer 65 may be separated from each other, such that the layers are discrete or, alternatively, these layers may be continuous, such that a layer runs continuously from the front belt end edge 55 to the back belt end edge 57. This may also be true for the front and back inner belt layers 66 and 67 - that is, they may also be longitudinally discrete or continuous. Further, the front and back outer belt layers 64 and 65 may be longitudinally continuous while the front and back inner belt layers 66 and 67 are longitudinally discrete, such that a gap is formed between them - a gap between the front and back inner and outer belt layers 64, 65, 66, and 67 is shown in Fig. 7 and a gap between the front and back inner belt layers 66 and 67 is shown in Fig. 8. In some configurations shown in Fig. 7 and 8, portions of the outer belt layers 64, 65 may be folded over onto the inner belt layers, respectively. In addition, as shown in Fig. 7, portions of the outer belt layers 64, 65 may also be folded over onto the chassis 52.

The front and back belts 54 and 56 may include slits, holes, and/or perforations providing increased breathability, softness, and a garment-like texture. Underwear-like appearance can be enhanced by substantially aligning the waist and leg edges at the side seams 58 (see Figs. 4 and 5).

The front and back belts 54 and 56 may comprise graphics (see e.g., 78 of Fig. 1). The graphics may extend substantially around the entire circumference of the absorbent article 10 and may be disposed across side seams 58 and/or across proximal front and back belt edges 15 and 17; or, alternatively, adjacent to the seams 58 and/or proximal front and back belt edges 15 and 17 in the manner described in U.S. Pat. No. 9,498, 389 to create a more underwear-like article. The graphics may also be discontinuous.

Alternatively, instead of attaching belts 54 and 56 to the chassis 52 to form a pant, discrete side panels may be attached to side edges of the chassis 22 and 24. Suitable forms of pants comprising discrete side panels are disclosed in U.S. Pat. Nos. 6,645,190; 8,747,379; 8,372,052; 8,361,048; 6,761,711; 6,817,994; 8,007,485; 7,862,550; 6,969,377; 7,497,851; 6,849,067; 6,893,426; 6,953,452; 6,840,928; 8,579,876; 7,682,349; 7,156,833; and 7,201,744.

### Topsheet

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured (Fig. 2, element 31), may have any suitable three-dimensional features, and/or may have a plurality of embossments (e.g., a bond pattern). The topsheet may comprise a variable basis weight nonwoven material, such as those described in U.S. Pat. Appl. No. 2017/0191198. The topsheet may be apertured by overbonding a material and then rupturing the overbonds through ring rolling, such as disclosed in U.S. Patent No. 5,628,097, to Benson et al., issued on May 13, 1997, and disclosed in U.S. Pat. Appl. Publication No. US 2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet. The topsheet may comprise a one or more layer hydroentangled material with or without apertures. The topsheet may comprise a variable basis weight nonwoven material.

### Backsheet

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials, such as films, which permit vapors to escape from the absorbent article, while still preventing, or at least inhibiting, bodily exudates from passing through the backsheet. The backsheet 28 may be coterminous with the outer cover material 40.

### Outer Cover Material

The outer cover material (sometimes referred to as a backsheet nonwoven) 40 may comprise one or more nonwoven materials joined to the backsheet 28 and that covers the backsheet 28. The outer cover material 40 forms at least a portion of the garment-facing surface 2 of the absorbent article 10 and effectively "covers" the backsheet 28 so that film is not present on the garment-facing surface 2. The outer cover material 40 may comprise a bond pattern, apertures, and/or three-dimensional features. The outer cover material 40 may be a hydroentangled nonwoven material or a variable basis weight nonwoven material. Such a material may have one or more layers.

### Absorbent Core

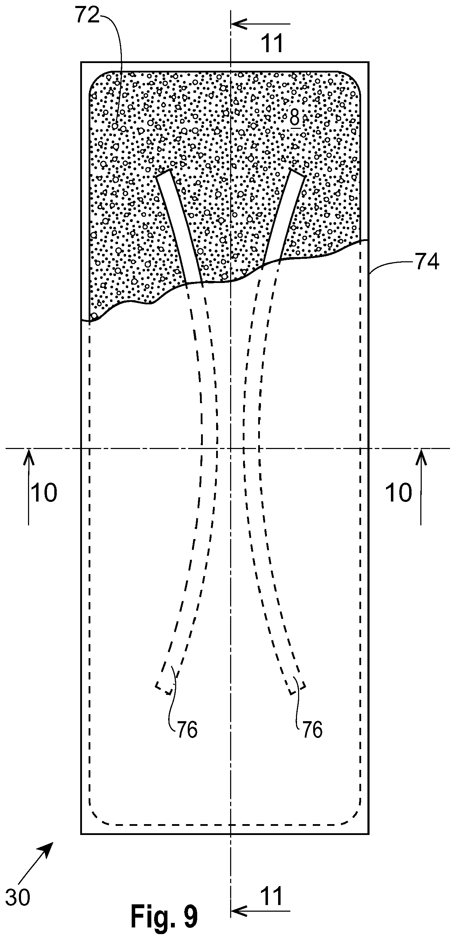

As used herein, the term "absorbent core" refers to a component of the absorbent article 10 for absorbing and containing liquid such as urine received by the absorbent article. The absorbent core thus typically has a high absorbent capacity. An example absorbent core 30 is schematically shown in Figs. 9-11. The absorbent core comprises an absorbent material 72. The absorbent core 30 may typically comprise a core wrap 74 that encloses or sandwiches the absorbent material.

The core wrap may be a single material that is folded and attached to itself, or it may comprise a separate top layer and bottom layer that may be bonded, adhesively joined, or otherwise joined together. The top and bottom layers of the core wrap may be the same or different. The absorbent material typically comprises superabsorbent particles which are optionally mixed with cellulose fibers. As used herein, "absorbent core" does not include any acquisition-distribution systems, topsheet, or backsheet of the absorbent article.

The example absorbent core 30 shown in isolation in Figs. 9-11 is in the dry state (before use). The absorbent core may typically have a generally rectangular shape as defined by its longitudinal edges and transversal front edge and back edge or may have other shapes.

Absorbent material 72 may be deposited as an absorbent layer having a generally rectangular outline, as represented in Fig. 9. A wide variety of absorbent cores may also be used. The absorbent material 72 layer may also have a non-rectangular perimeter ("shaped" core), in particular, the absorbent material 72 may define a tapering along its width towards the central region of the core (or "dog-bone" shape). In this way, the absorbent material deposition area may have a relatively narrow width in an area of the core intended to be placed in the crotch region of the absorbent article. This may provide for example better wearing comfort. Other shapes can also be used such as a "T" or "Y" or "X" or "L" or "U" or "O" or "hourglass" for the area of the absorbent material.

The absorbent material 72 may be any conventional absorbent material known in the art. For example, the absorbent material may comprise a blend of cellulose fibers and superabsorbent particles ("SAP"), typically with the percentage of SAP ranging from about 50% to about 75% by weight of the absorbent material. The absorbent material may also be free of cellulose fibers, as is known in so-called airfelt-free cores, where the absorbent material consists, or consists essentially, of SAP. The absorbent material may also be a high internal phase emulsion foam. High loft nonwoven materials may be present in the core bags, or proximate but outside the core bags. The absorbent material may comprise one or more layers of a coform material. The coform material may comprise a mixture of fibers and an absorbent material. The fibers may comprise staple fibers such as synthetic fibers or absorbent fibers. The synthetic fibers may comprise polypropylene fibers, polyethylene fibers, and/or bicomponent fibers. The absorbent fibers may comprise pulp, lyocell, and/or viscose. The fibers may also comprise thermoplastic filaments (scattered or interconnected networks). The absorbent material may comprise a super absorbent polymeric material in fiber or particle form. The one or more layers of coform material may comprise virgin or recycled materials. The staple fibers may be present in the coform material in an amount of from about 5wt. % to about 50wt.%. The absorbent material may be present in the nonwoven web in an amount of from about 50wt.% to about 95wt.%. The staple fibers may have an average length of from about 5 mm to about 50 mm. The staple fibers and absorbent fibers may be thermally bonded or hydraulically entangled to form the nonwoven web (as disclosed in WO2023/022979).

"Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of at least 20 g/g, in particular of from about 20 g/g to about 40 g/g. "Superabsorbent polymer particles", as used herein, refers to a superabsorbent polymer material which is in particulate form so as to be flowable in the dry state.

Various absorbent core designs comprising high amounts of SAP have been proposed in the past, see for example in U.S. Pat. No. 5,599,335 (Goldman), EP1,447,066 (Busam), WO95/11652 (Tanzer), U.S. Pat. Appl. Pub. No. 2008/0312622A1 (Hundorf), WO2012/052172 (Van Malderen). In particular, the SAP printing technology as disclosed in U.S. Pat. No. 7,838,722 (Blessing), U.S. Pat. No. 9,072,634 and U.S. Pat. No. 8,206,533 (both to Hundorf et al.) may be used. The present disclosure however is not limited to a particular type of absorbent core. The absorbent core may also comprise one or more glues such as an auxiliary glue applied between the internal surface of one (or both) of the core wrap layers and the absorbent material to reduce leakage of SAP outside the core wrap. A micro-fibrous adhesive net may also be used in air-felt free cores as described in the above Hundorf references. These glues are not represented in the Figures for simplicity. Other core constructions comprising a high loft nonwoven substrate, such as a carded nonwoven layer, having a porous structure into which SAP particles have been deposited, may also be used in present disclosure.

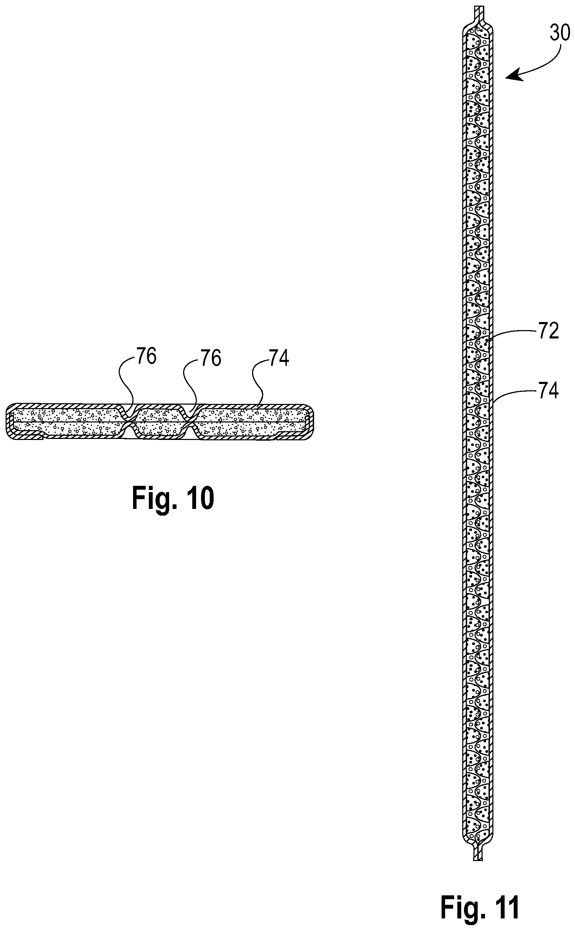

The absorbent material may be deposited as a continuous layer within the core wrap. The absorbent material may also be present discontinuously, for example, as individual pockets or stripes of absorbent material enclosed within the core wrap and separated from each other by material-free junction areas. A continuous layer of absorbent material, in particular of SAP, may also be obtained by combining two absorbent layers having matching discontinuous absorbent material application pattern, wherein the resulting layer is substantially continuously distributed across the absorbent particulate polymer material area, as illustrated in Figs. 10-11. As for example taught in U.S. Pat. Appl. Pub. No. 2008/0312622A1 (Hundorf), each absorbent material layer may thus comprise a pattern having absorbent material land areas and absorbent material-free junction areas, wherein the absorbent material land areas of the first layer correspond substantially to the absorbent material-free junction areas of the second layer and vice versa.

The basis weight (amount deposited per unit of surface) of the absorbent material may also be varied to create a profiled distribution of absorbent material, in particular in the longitudinal direction to provide more absorbency towards the center and the middle of the core, but also in the transversal direction, or both directions of the core. The absorbent core may also comprise one or more longitudinally (or otherwise) extending channels 76, which are areas of the absorbent layer substantially free of absorbent material within the absorbent material layer. The top side of the core wrap may be advantageously bonded to the bottom side of the core by adhesive, mechanical or ultrasonic bonding through these material-free areas. Example disclosures of such channels in an airfelt-free core can be found in U.S. Pat. No. 9,789,011. One or more channels may also be formed in absorbent cores comprising a mix of cellulose fibers and SAP particles. These channels may embody any suitable shapes and any suitable number of channels may be provided. In other instances, the absorbent core may be embossed to create the impression of channels, with absorbent material remaining within the channels. The absorbent core in Figs. 9-11 is merely an example absorbent core. Many other absorbent cores with or without channels are also within the scope of the present disclosure.

### Barrier Leg Cuffs/Leg Elastics

Referring to Figs. 1 and 2, for example, the absorbent article 10 may comprise one or more pairs of barrier leg cuffs 32 and one or more pairs of leg elastics 34. The barrier leg cuffs 32 may be positioned laterally inboard of leg elastics 34. Each barrier leg cuff 32 may be formed by a piece of material which is bonded to the absorbent article 10 so it can extend upwards from a wearer-facing surface 4 of the absorbent article 10 and provide improved containment of body exudates approximately at the junction of the torso and legs of the wearer. The barrier leg cuffs 32 are delimited by a proximal edge joined directly or indirectly to the topsheet and/or the backsheet and a free terminal edge, which is intended to contact and form a seal with the wearer's skin. The barrier leg cuffs 32 may extend at least partially between the front end edge 18 and the back end edge 20 of the absorbent article 10 on opposite sides of the central longitudinal axis 50 and may be at least present in the crotch region 14. The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front end edge 18 and the back end edge 20. The leg elastics 34 essentially cause portions of the absorbent article 10 proximate to the chassis side edges 22, 24 to help form a seal around the legs of the wearer. The leg elastics 34 may extend at least within the crotch region 14. The barrier leg cuffs 32 may comprise one or more layers of nonwoven material. The nonwoven material may be hydroentangled.

### Elastic Waistband

Referring to Figs. 1 and 2, the absorbent article 10 may comprise one or more elastic waistbands 36. The elastic waistbands 36 may be positioned on the garment-facing surface 2 or the wearer-facing surface 4. Alternatively, the elastic waistbands may be positioned intermediate the topsheet and the backsheet. As an example, a first elastic waistband 36 may be present in the front waist region 12 near the front end edge 18 and a second elastic waistband 36 may be present in the back waist region 16 near the back end edge 20. The elastic waistbands 36 may aid in sealing the absorbent article 10 around a waist of a wearer and at least inhibiting bodily exudates from escaping the absorbent article 10 through the waist opening circumference. In some instances, an elastic waistband may fully surround the waist opening circumference of an absorbent article. The elastic waistband may comprise an elastic film joined to the topsheet and a nonwoven material covering the elastic film. In other instances, the elastic waistband may comprise an elastic film sandwiched between two nonwoven materials. The elastic film may be ultrasonically bonded, or otherwise bonded or attached, to the one or more nonwoven materials. The one or more nonwoven materials may be hydroentangled. The elastic film and/or the nonwoven materials may be preactivated (i.e., activated prior to being joined together) or the formed elastic film/nonwoven laminate may be activated post laminate formation.

### Acquisition and distribution system

Referring to Figs. 1, 2, 7, and 8, an acquisition and distribution system 38 may be present at least partially intermediate the topsheet 26 and the absorbent core 30. The acquisition and distribution system 38 comprises acquisition materials, which are typically hydrophilic materials that provide significant wicking of bodily exudates. These materials may dewater the topsheet 26 and quickly move bodily exudates into the absorbent core 30. The acquisition materials 38 may comprise one or more nonwoven materials, foams, formed films, apertured formed films, cellulosic materials, cross-linked cellulosic materials, air laid cellulosic nonwoven materials, spunlace materials, or combinations thereof, for example, acquisition and distribution system 38 may be rectangular or may be shaped, such as hourglass or dogbone shaped. Typically, an acquisition and distribution system 38 may have a width and length that are smaller than the width and length of the topsheet 26. The acquisition and distribution system 38 may have one or more channels as described above with reference to the absorbent core 30 (including the embossed version). The channels in the acquisition and distribution system 38 may align or not align with channels in the absorbent core 30. In an example, a first acquisition material may comprise a nonwoven material and as second acquisition material may comprise a cross-linked cellulosic material. In an example, a first layer of acquisition and distribution system 38 may comprise a nonwoven material and as second layer of the acquisition and distribution system 38 may comprise a cross-linked cellulosic material. The second layer of the acquisition and distribution system 38 may be provided between the first layer of acquisition and distribution system 38 and the absorbent core. The first layer of acquisition and distribution system 38 may be provided between the topsheet and the second layer of the acquisition and distribution system 38.

The acquisition and distribution system 38 comprises a front waist edge 212 in the front waist region 12 and a back waist edge 216 in the back waist region 16. The front waist edge of the acquisition and distribution system 212 corresponds to the line, which is perpendicular to the central longitudinal axis 50 and drawn through the point of the acquisition and distribution system 38 closest to the front end edge 18. The back waist edge of the acquisition and distribution system 216 corresponds to the line, which is perpendicular to the central longitudinal axis 50 and drawn through the point of the acquisition and distribution system 38 closest to the back end edge 20. The acquisition and distribution system 38 has a central transverse axis 248, which bisects the acquisition and distribution system 38 into two parts with equal length - corresponding to the distance of the front waist edge 212 to the central transverse axis 248, to the distance of the back a waist edge 216 to the central transverse axis 248 respectively - in the direction of the central longitudinal axis 50 of the absorbent article 10. The central transverse axis of the acquisition and distribution system 248 extends perpendicular to the central longitudinal axis 50 of the absorbent article 10.

### Landing Zone

Referring to Figs. 1 and 2, the absorbent article 10 may have a landing zone area 44 that is formed in a portion of the garment-facing surface 2 of the outer cover material 40. The landing zone area 44 may be in the back waist region 16 if the absorbent article 10 fastens from front to back or may be in the front waist region 12 if the absorbent article 10 fastens back to front. In some instances, the landing zone 44 may be or may comprise one or more discrete nonwoven materials that are attached to a portion of the outer cover material 40 in the front waist region 12 or the back waist region 16 depending upon whether the absorbent article fastens in the front or the back. In essence, the landing zone 44 is configured to receive the fasteners 46 and may comprise, for example, a plurality of loops configured to be engaged with, a plurality of hooks on the fasteners 46, or *vice versa.*

### Wetness Indicator/Graphics

Referring to Fig. 1, the absorbent articles 10 of the present disclosure may comprise graphics 78 and/or wetness indicators 80 that are visible from the garment-facing surface 2. The graphics 78 may be printed on the landing zone 40, the backsheet 28, and/or at other locations. The wetness indicators 80 are typically applied to the absorbent core facing side of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78.

### Front and Back Ears

Referring to Figs. 1 and 2, as referenced above, the absorbent article 10 may have front and/or back ears 47, 42 in a taped diaper context. Only one set of ears may be required in most taped diapers. The single set of ears may comprise fasteners 46 configured to engage the landing zone or landing zone area 44. If two sets of ears are provided, in most instances, only one set of the ears may have fasteners 46, with the other set being free of fasteners. Any ears with fasteners may be configured to engage an opposing ear or the landing zone or landing zone area 44. One set of ears may comprise primary fasteners and the other set of ears may comprise secondary fasteners. The ears, or portions thereof, may be elastic or may have elastic panels. In an example, an elastic film or elastic strands may be positioned intermediate a first nonwoven material and a second nonwoven material. One or more of the first and second nonwoven materials may be hydroentangled. The elastic film may or may not be apertured. The ears may be shaped. The ears may be integral (e.g., extension of the outer cover material 40, the backsheet 28, and/or the topsheet 26) or may be discrete components attached to a chassis 52 of the absorbent article on a wearer-facing surface 4, on the garment-facing surface 2, or intermediate the two surfaces 4, 2. The back ears may comprise an elastic film sandwiched between two nonwoven materials forming a laminate. The elastic film and/or the nonwoven materials may be pre-activated (i.e., activated prior to being joined together) and the laminate may be joined by ultrasonic bonds. Such laminates are disclosed in U.S. Pat. No. 10,485,713. In other instances, the elastic film may not be preactivated and instead the laminate may be activated after the laminate is formed. Such laminates may also be ultrasonically bonded.

### Masking Layer

One or more masking layers or materials may be provided in the absorbent articles 10. A masking layer may be a layer that provides a cushiony feel when the absorbent article is touched from the garment-facing surface 2 or the wearer-facing surface 4. The masking layer may "mask" a grainy feel potentially caused by the absorbent material 72, such as superabsorbent polymers. The masking layer may "mask" bodily exudates from being visible when viewing the wearer-facing surface 4 or the garment-facing surface 2 of the absorbent article 10. The masking layer may have a basis weight in the range of about 15 gsm to about 50 gsm or about 15 gsm to about 40 gsm. The masking layer may comprise one or more nonwoven materials (e.g., a hydroentangled nonwoven material), foams, pulp layers, and/or other suitable materials. The masking layer may be the outer cover material 40. The masking layer may be the layer forming the garment-facing side or the wearer-facing side of the core bag 74. The masking layer may be a separate material positioned intermediate the garment-facing side of the core bag 74 and the liquid impermeable backsheet 28.

### Arrays

"Array" means a display of packages comprising disposable absorbent articles of different article constructions (e.g., different elastomeric materials [compositionally and/or structurally] in the side panels, back ears, side flaps and/or belts flaps, different graphic elements, different product structures, fasteners, waistbands, or lack thereof). The packages may have the same brand and/or sub-brand and/or the same trademark registration and/or having been manufactured by or for a common manufacturer and the packages may be available at a common point of sale (e.g. oriented in proximity to each other in a given area of a retail store). An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Huggies," and same sub-brand, for example, "Pull-Ups." A different product in the array may have the same brand "Huggies" and the sub-brand "Little Movers." The differences between the "Pull-Ups" product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag.

Further regarding "Arrays," as another example an array may be formed by different products having different product forms manufactured by the same manufacturer, for example, "Kimberly-Clark", and bearing a common trademark registration for example, one product may have the brand name "Huggies," and sub-brand, for example, "Pull-Ups." A different product in the array may have a brand/sub-brand "Good Nites" and both are registered trademarks of The Kimberly-Clark Corporation and/or are manufactured by Kimberly-Clark. Arrays also often have the same trademarks, including trademarks of the brand, sub-brand, and/or features and/or benefits across the line-up. "On-line Array" means an "Array" distributed by a common on-line source.

### Fasteners

The back ears 42 may comprise fasteners 46. The fasteners 46 may be configured to cooperate with the landing zone area or landing zone material 44 on the garment-facing surface of the front waist region 12 of the absorbent article 10. Additionally, the absorbent article may comprise one or more secondary fasteners 49. The secondary fasteners 49 may be disposed on the outer cover material 40 or a component of the absorbent article, such as the landing zone. The secondary fasteners may be a separate material joined to the absorbent article. The secondary fasteners may be integrally formed from a material of the absorbent article. For example, the secondary fasteners may comprise one or more hooks or protrusions formed from the material of the outer cover material 40, the landing zone material 44, or a film. The hooks or protrusions may be formed using the process described in U.S. Pat. No. 8,784,722 to Rocha et al. The secondary fasteners may be configured to cooperate with a portion of the back ears. For example, a secondary fastener comprising hooks or protrusions are configured to engage the nonwoven material of the back ear. It is to be appreciated that the secondary fasteners may be any mechanical fastener that is configured to cooperate with a component of the absorbent article.

### Bio-Based Content for Components

Components of the absorbent articles described herein may at least partially be comprised of bio-based content as described in U.S. Pat. Appl. No. 2007/0219521A1. For example, the superabsorbent polymer component may be bio-based via their derivation from bio-based acrylic acid. Bio-based acrylic acid and methods of production are further described in U.S. Pat. Appl. Pub. No. 2007/0219521 and U.S. Pat. Nos. 8,703,450; 9,630,901 and 9,822,197. Other components, for example nonwoven and film components, may comprise bio-based polyolefin materials. Bio-based polyolefins are further discussed in U.S. Pat. Appl. Pub. Nos. 2011/0139657, 2011/0139658, 2011/0152812, and U.S. Pat. Nos. 10,166,312 and 9,169,366. Example bio-based polyolefins for use in the present disclosure comprise polymers available under the designations SHA7260^{™}, SHE150^{™}, or SGM9450F^{™} (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

### Recycle Friendly and Bio-Based Absorbent Articles

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in U.S. Pat. No. 11,433,158, issued on September 6, 2022.

### Examples

Example 1 and Comparative Example 1 contained Pampers Baby Dry diapers, Size 5, as commercially available in Germany in October 2022, in a total count of 30. The taped diapers comprised an airfelt-free core and an acquisition and distribution system. Both in Example 1 and Comparative Example 1, the diapers were aligned in a single stack. The diapers, the diaper stack respectively, were compressed at the same compression levels for Example 1 and Comparative Example 1 with pressure being applied in the in-stack direction. The package material for both examples corresponded to the polyethylene film material as employed in the packages of Pampers Baby Dry diapers, Size 5, Single Pack as commercially available in Germany in October 2022.

Comparative Example 1 contained diapers which were bi-folded around the central lateral axis. Thus, the diapers were in the bi-folded configuration as present in Pampers Baby Dry diapers, Size 5, Single Pack as commercially available in Germany in October 2022. The bi-folded diapers had an average length in the longitudinal direction of 253.5 mm.

Example 1 contained diapers in a folded configuration according to the invention. The first fold line substantially corresponded to the central transverse axis of the acquisition and distribution system and the second fold line substantially corresponded to the front and back waist edge of the acquisition and distribution system. Thus, the first fold line was located 39.5 mm closer to the front end edge than to the back end edge, and the second fold line had a distance of 60 mm from the front end edge and of 139 mm from the back end edge. The distances are averaged for the total count of diapers. The folded diapers had an average length in the longitudinal direction of 154 mm.

Example 1 and Comparative Example 1 were subjected to the Package Compression Test.

**Table 1: Results for Example 1 and Comparative Example 1**

| | **Example 1** | **Comp. Example 1** |
|---|---|---|
| Package Out-of-Stack Waist Compression Deflection at 0.6 psi [mm] | 2.03 | 4.70 |
| Package Out-of-Stack Compression Deflection Differential at 0.6 psi [mm] | 0.23 | 3.24 |
| Package Out-of-Stack Waist Compression Deflection at 5.0 psi [mm] | 7.6 | 16.7 |
| Package Out-of-Stack Compression Deflection Differential at 5.0 psi [mm] | -3.9 | 8.6 |
| Package Out-of-Stack Waist Compression Deflection Factor [mm/psi] | 1.16 | 2.44 |
| Package Out-of-Stack Compression Deflection Factor Differential [mm/psi] | -1.06 | 0.95 |
| Package In-Stack Waist Compression Deflection Factor [mm/psi] | 2.86 | 4.55 |
| Package In-Stack Compression Deflection Factor Differential [mm/psi] | -0.27 | 1.69 |
| Package In-Stack Waist Compression Deflection at 0.6 psi [mm] | 1.51 | 2.87 |
| Package In-Stack Compression Deflection Differential at 0.6 psi [mm] | -0.17 | 1.18 |

The results show that Example 1 exhibits a much more uniform pressure response and much less pronounced deflection than Comparative Example 1, both from different locations of the sides and/ or top and bottom of the packages.

### Test Methods

### Package Compression Test

In the Package Compression Test, packages of stacked absorbent articles are interrogated in a controlled way using a tensile tester, and the load-displacement response at specific locations is used to establish the package displacement at applied pressure of interest and/or the linear factor relating package displacement and applied pressure over a pressure range of interest. All laboratory work is carried out at 23 ± 2 °C and at 50 ± 5 %RH on specimen packages that have been equilibrated to these same conditions for at least two hours.

### Apparatus

For all measurements in this method, a constant-rate-of strain tensile tester is used. The upper, moving crosshead is outfitted with a 100-N load cell and a flat, circular probe 40.0 mm in diameter. On the lower, stationary crosshead is mounted a horizontal, rigid flat plate large enough to support the entire package under interrogation. Heavy blocks may optionally be placed on either side of the package to hold it vertical at the start of compression.

The tensile tester is operated in compression mode. The measurement is started with the upper, moving probe in minimal contact with the specimen package (generally less than 0.1 N of load). The probe proceeds in compression mode, moving downward at 0.5 mm/s until 50 N of load is achieved, at which point that probe returns to the position at which 0.05 N is first achieved and the acquisition terminates. Data are recorded at 100 Hz or greater frequency. Only data on the compression stroke (and not the return stroke) are used subsequently in this method.

### Specimen description and data acquisition

For any sample absorbent article package of interested, five like specimens of packages are used to establish load-displacement curves in four positions. Packages generally have the shape of a rectangular solid and can be described with three mutually perpendicular axes. For the purpose of describing specimen orientation in this method, the "longitudinal axis" of a package corresponds to the axis of a package that is generally parallel to the longitudinal axes of contained folded absorbent articles. "Lateral axis" of a package corresponds to the axis of a package that is generally parallel to the lateral axes of the contained folded absorbent articles. "Stack axis" of a package corresponds to the axis of a package that is generally parallel to the axis along which articles are stacked in the package.

For each specimen interrogated, load-displacement curves are measured at four locations and package positions, or measurement orientations.

To measure the "Out-of-Stack-Waist" load-displacement curve, the specimen package is positioned on the lower plate of the tensile tester so that the package longitudinal axis is vertical. The surface of the package corresponding to the folds in the crotch regions of the contained articles is flush against the lower plate. The 40-mm upper compression probe is positioned to be centered along both stack and lateral package axes on the upward facing surface of the package, which corresponds to the waist regions of the contained articles.

To measure the "Out-of-Stack-Crotch" load-displacement curve, the specimen package is positioned on the lower plate of the tensile tester so that the package longitudinal axis is vertical. The surface of the package corresponding to the waist regions of the contained articles is flush against the lower plate. The 40-mm upper compression probe is positioned to be centered along both stack and lateral package axes on the upward facing surface of the package, which corresponds to the folds in the crotch regions of the contained articles.

To measure the "In-Stack-Waist" load-displacement curve the specimen package is positioned on the lower plate of the tensile tester so that the package stack axis is vertical. The 40-mm upper compression probe is positioned to be centered along the lateral package axes on the upward facing surface of the package and is positioned so as to be centered at a point 50 ± 5 mm from the edge of the package that corresponds to the waist regions of the contained articles.

To measure the "In-Stack-Crotch" load-displacement curve the specimen package is positioned on the lower plate of the tensile tester so that the package stack axis is vertical. The 40-mm upper compression probe is positioned to be centered along the lateral package axes on the upward facing surface of the package and is positioned so as to be centered at a point 50 ± 5 mm from the edge of the package that corresponds to the folds in the crotch regions of the contained articles.

While these four orientations are described for a sample package containing a single stack of absorbent articles, these orientations may be generalized for packages containing multiple stacks of articles to interrogate the properties of individual stacks and/or the overall package properties resulting from multiple stacks.

### Data analysis and calculation of raw parameters

For each load displacement curve recorded (for each specimen and for each of the positions described), the following processing and raw parameter definition is performed. First, load data are converted to pressure pounds per square inch (psi) based on the measured load and area of the flat face of the 40-mm diameter upper probe. Displacement data are zeroed at the point at which measured pressure first achieves 0.1 psi such that all displacement data are relative to the dimension at 0.1 psi. The pressure-displacement curves are then used to define three raw parameters. The absolute displacement in millimeters (mm) at 0.6 psi is recorded as the 0.6-psi displacement. The absolute displacement in millimeters (mm) at 5.0 psi is recorded as the 5.0-psi displacement. The slope of the pressure-displacement curve between 1.0 and 5.0 psi is fitted linearly using least squares, and this is recorded as the slope. As a final step, for each specimen position, the raw parameter outputs measured above are averaged (arithmetic mean) among the five constituent specimen raw output. So for each of the four package positions measured for the sample package of interest, there is a resulting raw parameter 0.6-psi displacement, a raw parameter 5.0-psi displacement, and a raw parameter slope.

### Output parameter definition and calculation

Output parameters are in units of mm or mm/psi and are reported with two decimal places of precision. Output parameters are defined as follows:
Package Out-of-Stack Waist Compression Deflection at 0.6 psi is the 0.6-psi displacement of the package in the Out-of-Stack-Waist measurement orientation. It is reported in mm to the nearest 0.01 mm.
Package Out-of-Stack Compression Deflection Differential at 0.6 psi is the difference between the 0.6-psi displacement of the package in the Out-of-Stack-Waist measurement orientation and the 0.6-psi displacement of the package in the Out-of-Stack-Crotch measurement orientation. It is reported in mm to the nearest 0.01 mm.
Package Out-of-Stack Waist Compression Deflection at 5.0 psi is the 5.0-psi displacement of the package in the Out-of-Stack-Waist measurement orientation. It is reported in mm to the nearest 0.01 mm.
Package Out-of-Stack Compression Deflection Differential at 5.0 psi is the difference between the 5.0-psi displacement of the package in the Out-of-Stack-Waist measurement orientation and the 0.6-psi displacement of the package in the Out-of-Stack-Crotch measurement orientation. It is reported in mm to the nearest 0.01 mm.
Package Out-of-Stack Waist Compression Deflection Factor is the inverse of the slope in the Out-of-Stack-Waist measurement orientation. It is reported in mm/psi to the nearest 0.01 mm/psi.
Package Out-of-Stack Compression Deflection Factor Differential is the difference between the inverse of the slope in the Out-of-Stack-Waist measurement orientation and the inverse of the slope in the Out-of-Stack-Crotch measurement orientation. It is reported in mm/psi to the nearest 0.01 mm/psi.
Package In-Stack Waist Compression Deflection Factor is the inverse of the slope in the In-Stack-Waist measurement orientation. It is reported in mm/psi to the nearest 0.01 mm/psi.
Package In-Stack Compression Deflection Factor Differential is the difference between the inverse of the slope in the In-Stack-Waist measurement orientation and the inverse of the slope in the In-Stack-Crotch measurement orientation. It is reported in mm/psi to the nearest 0.01 mm/psi.
Package In-Stack Waist Compression Deflection at 0.6 psi is the 0.6-psi displacement of the package in the In-Stack-Waist measurement orientation. It is reported in mm to the nearest 0.01 mm.
Package In-Stack Compression Deflection Differential at 0.6 psi is the difference between the 0.6-psi displacement of the package in the In-Stack-Waist measurement orientation and the 0.6-psi displacement of the package in the In-Stack-Crotch measurement orientation. It is reported in mm to the nearest 0.01 mm.

### Caliper Test Method

The caliper, or thickness, of a single-layer test sample is measured under a static load by a micrometer, in accordance with compendial method ISO 534, with modifications noted herein. All measurements are performed in a laboratory maintained at 23 °C ± 2 C° and 50% ± 2% relative humidity and test samples are conditioned in this environment for at least 2 hours prior to testing.

Caliper is measured with a micrometer equipped with a pressure foot capable of exerting a steady pressure of 70 kPa ± 0.05 kPa onto the test sample. The micrometer is a dead-weight type instrument with readings accurate to 0.1 micron. A suitable instrument is the TMI Digital Micrometer Model 49-56, available from Testing Machines Inc., New Castle, DE, or equivalent. The pressure foot is a flat ground circular movable face with a diameter that is smaller than the test specimen and capable of exerting the required pressure. A suitable pressure foot has a diameter of 16.0 mm. The test sample is supported by a horizontal flat reference platform that is larger than and parallel to the surface of the pressure foot. The system is calibrated and operated per the manufacturer's instructions.

Measurements are made on single-layer test samples taken from rolls or sheets of the raw material, or test samples obtained from a finished package. When excising the test sample from a finished package, use care to not impart any contamination or distortion to the sample during the process. The excised sample should be free from residual adhesive and taken from an area of the package that is free from any seams or folds. The test sample is ideally 200 mm2 and must be larger than the pressure foot.

To measure caliper, first zero the micrometer against the horizontal flat reference platform. Place the test sample on the platform with the test location centered below the pressure foot. Gently lower the pressure foot with a descent rate of 3.0 mm per second until the full pressure is exerted onto the test sample. Wait 5 seconds and then record the caliper of the test sample to the nearest 0.1 micron. In like fashion, repeat for a total of ten replicate test samples. Calculate the arithmetic mean for all caliper measurements and report the value as Caliper to the nearest 0.1 micron.

### Basis Weight Test Method

The basis weight of a test sample is the mass (in grams) per unit area (in square meters) of a single layer of material and is measured in accordance with compendial method ISO 536. The mass of the test sample is cut to a known area, and the mass of the sample is determined using an analytical balance accurate to 0.0001 grams. All measurements are performed in a laboratory maintained at 23 °C ± 2 C° and 50% ± 2% relative humidity and test samples are conditioned in this environment for at least 2 hours prior to testing.

Measurements are made on test samples taken from rolls or sheets of the raw material, or test samples obtained from a finished package. When excising the test sample from a finished package, use care to not impart any contamination or distortion to the sample during the process. The excised sample should be free from residual adhesive and taken from an area of the package that is free from any seams or folds. The test sample must be as large as possible so that any inherent material variability is accounted for.

Measure the dimensions of the single layer test sample using a calibrated steel metal ruler traceable to NIST, or equivalent. Calculate the Area of the test sample and record to the nearest 0.0001 square meter. Use an analytical balance to obtain the Mass of the test sample and record to the nearest 0.0001 gram. Calculate Basis Weight by dividing Mass (in grams) by Area (in square meters) and record to the nearest 0.01 grams per square meter (gsm). In like fashion, repeat for a total of ten replicate test samples. Calculate the arithmetic mean for Basis Weight and report to the nearest 0.01 grams/square meter.

### In-Bag Stack Height Test Method

The in-bag stack height of a package of absorbent articles is determined as follows: Equipment

A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within ± 0.5 mm. The horizontal sliding plate and the horizontal base plate are larger than the surface of the absorbent article package that contacts each plate, i.e., each plate extends past the contact surface of the absorbent article package in all directions. The horizontal sliding plate exerts a downward force of 850 ± 1 gram-force (8.34 N) on the absorbent article package, which may be achieved by placing a suitable weight on the center of the non-package-contacting top surface of the horizontal sliding plate so that the total mass of the sliding plate plus added weight is 850 ± 1grams.

### Test Procedure

Absorbent article packages are equilibrated at 23 ± 2 °C and 50 ± 5 % relative humidity prior to measurement.

The horizontal sliding plate is raised, and an absorbent article package is placed centrally under the horizontal sliding plate in such a way that the absorbent articles within the package are in a horizontal orientation. Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within ± 0.5 mm ten seconds after releasing the horizontal sliding plate. Five identical packages (same size packages and same absorbent articles counts) are measured, and the arithmetic mean is reported as the package width. The "In-Bag Stack Height" = (package width/absorbent article count per stack) * 10 is calculated and reported to within ±0.5 mm.

### Contemplated Examples

A1.An absorbent article (10) in the form of diapers and/ or pants comprising
   a liquid permeable topsheet (26),
   a liquid impermeable backsheet (28), and
   an absorbent core (30) at least partially positioned between the topsheet (26) and the backsheet (28);
   a front waist region (12), a back waist region (16), a crotch region (14) located between the front waist region (12) and the back waist region (16);
   a central longitudinal axis (50);
   a first fold (400) extending transversely across the crotch region (14) along a first fold line, which is substantially perpendicular to the central longitudinal axis (50); and further comprising a second fold (500) extending transversely across the front and back waist regions (12, 16) along a second fold line, which is substantially perpendicular to the central longitudinal axis (50);
   wherein the second fold line lies between the first fold line and a front waist edge of the absorbent core (112) in the front waist region (12) and/ or lies between the first fold line and a back waist edge of the absorbent core (116) in the back waist region (16).
A2.The absorbent article (10) of Example A1, wherein the absorbent article (10) further comprises an acquisition and distribution system (38) at least partially positioned intermediate the absorbent core (30) and the topsheet (26) and wherein the crotch region (14) at least partially comprises the acquisition and distribution system (38).
A3.The absorbent article (10) of Example A2, wherein the second fold line
   either corresponds to a front waist edge of the acquisition and distribution system (212) or lies between the front waist edge of the acquisition and distribution system (212) and the front waist edge of the absorbent core (112) in the front waist region (12); and
   either corresponds to a back waist edge of the acquisition and distribution system (216) or lies between the front waist edge of the acquisition and distribution system (212) and the front waist edge of the absorbent core (112) in the front waist region (12) and/ or lies between the back waist edge of the acquisition and distribution system (216) and the back waist edge of the absorbent core (116) in the back waist region (16).
A4.The absorbent article (10) of Examples A2 or A3, wherein the acquisition and distribution system (38) has a non-uniformly distributed basis weight at least in the direction of the longitudinal axis (50).
A5.The absorbent article (10) of Example A4, wherein the acquisition and distribution system (38) has its maximum basis weight in the crotch region (14) and the basis weight decreases in the direction of the longitudinal axis (50) towards the front waist edge of the acquisition and distribution system (212) and/ or the back waist edge of the acquisition and distribution system (216).
A6.The absorbent article (10) of any one of Examples A1 to A5, wherein the first fold line substantially bisects the acquisition and distribution system (38) into two halves of substantially equal length in the direction of the central longitudinal axis (50).
A7.The absorbent article (10) of any one of Examples A1 to A6, wherein the backsheet (28) comprises a breathable film.
A8.The absorbent article (10) of any one of Examples A1 to A7, wherein the absorbent core (30) comprises absorbent material (72) comprising a blend of cellulose fibers and superabsorbent particles; and/ or high internal phase emulsion foam and wherein the absorbent material (72) is non-uniformly distributed at least in the direction of the longitudinal axis (50).
A9.The absorbent article (10) of Example A8, wherein the absorbent material (72) has its maximum basis weight in the crotch region (14) and the basis weight decreases in the direction of the longitudinal axis (50) towards the front waist edge of the absorbent core (112) and/ or the back waist edge of the absorbent core (116).
A10. The absorbent article (10) of any one of Examples A1 to A9, wherein absorbent article (10) the first fold line divides the crotch region (14) into two parts of unequal length in the direction of the central longitudinal axis (50).
A11. The absorbent article (10) of Examples A1 to A10, wherein the first fold line is closer to the front-end edge (18) than to the back-end edge (20) of the absorbent article (10).
A12. The absorbent article (10) of Example A10 or Example A11, wherein the absorbent article (10) comprises back ears (42) in the back waist region (16).
A13. The absorbent article (10) of any one of Examples A1 to A9, wherein the first fold line substantially bisects the crotch region (14) into two halves of substantially equal length in the direction of the central longitudinal axis (50).
A14. The absorbent article (10) of Example A13, wherein the absorbent article (10) is a pant-type article.
A15. The absorbent article (10) of any one of Examples A1 to A14, wherein the absorbent core (30) comprises less than 5% by weight of cellulose, more preferably less than 2% and most preferably is cellulose free.
A16. The absorbent article (10) of any one of Examples A1 to A15, wherein absorbent article (10) comprises one or more channels (76), wherein the second fold line lies between the front waist edge of the one or more channels (76) and the front waist edge of the absorbent core (112) in the front waist region (12) and/ or between the back waist edge of the one or more channels (76) and the back waist edge of the absorbent core (116) in the back waist region (16).
A17. The absorbent article (10) of any one of Examples A1 to A16, wherein the absorbent article (10) further comprises an elastic waistband (36).
B1.A package (1) comprising a plurality of absorbent articles (10) in the form of diapers and/ or pants,
   the package (1) comprising a flexible package material,
   wherein each of the plurality of absorbent articles (10) comprises
      a liquid permeable topsheet (26),
      a liquid impermeable backsheet (28), and
      an absorbent core (30) at least partially positioned between the topsheet (26) and the backsheet (28);
      a front waist region (12), a back waist region (16), a crotch region (14) located between the front waist region (12) and the back waist region (16);
      a central longitudinal axis (50);
      a first fold (400) extending transversely across the crotch region (14) along a first fold line, which is substantially perpendicular to the central longitudinal axis (50); and
      a second fold (500) extending transversely across the front and back waist regions (12, 16) along a second fold line, which is substantially perpendicular to the central longitudinal axis (50);
   wherein the package (1) has a Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm according to the Package Compression Test detailed herein.
B2. The package of Example B1, wherein the package (1) has a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm according to the Package Compression Test detailed herein.
B3. The package of any one of Examples B1 or B2, wherein the package (1) has a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm according to the Package Compression Test detailed herein.
B4. The package of any one of Examples B1 to B3, wherein the package (1) has a Package Out-of-Stack Compression Deflection Differential at 5.0 psi of at most 8.0 mm, preferably at most 7.0 mm, more preferably at most 6.0 mm, even more preferably at most 5.0 mm, most preferably at most 4.0 mm or even at most 3.0 mm according to the Package Compression Test detailed herein.
B5. The package of any one of Examples B1 to B4, wherein the package (1) has a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi according to the Package Compression Test detailed herein.
B6. The package of any one of Examples B1 to B5, wherein the package (1) has a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi according to the Package Compression Test detailed herein.
B7. The package of any one of Examples B1 to B6, wherein the package (1) has a Package In-Stack Waist Compression Deflection Factor of at most 4.25 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi according to the Package Compression Test detailed herein.
B8. The package of any one of Examples B1 to B7, wherein the package (1) has a Package In-Stack Compression Deflection Factor Differential of at most 1.50 mm/ psi, preferably at most 1.25 mm/ psi, more preferably at most 1.00 mm/ psi, even more preferably at most 0.75 mm/ psi, most preferably at most 0.50 mm/ psi or even at most 0.25 mm/ psi according to the Package Compression Test detailed herein.
B9. The package of any one of Examples B1 to B8, wherein the package (1) has a Package In-Stack Waist Compression Deflection at 0.6 psi of at most 2.75 mm, preferably at most 2.50 mm, more preferably at most 2.25 mm, even more preferably at most 2.00 mm, most preferably at most 1.75 mm or even at most 1.50 mm according to the Package Compression Test detailed herein.
B10. The package of any one of Examples B1 to B9, wherein the package (1) has a Package In-Stack Compression Deflection Differential at 0.6 psi of at most 1.00 mm, preferably at most 0.80 mm, more preferably at most 0.60 mm, even more preferably at most 0.40 mm, most preferably at most 0.30 mm or even at most 0.20 mm according to the Package Compression Test detailed herein.
B11. The package of any one of Examples B1 to B10, wherein the package has an in-bag stack height of from 70 mm to 110 mm, according to the In-Bag Stack Height Test detailed herein.
B12. The package of any one of Examples B1 to B11, wherein the package has a Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein.
B13. The package of any of Examples B1 to B12, wherein the flexible package material comprises one or more materials selected from the group consisting of polymeric film, paper, paper based material, paper with one or more barrier layers, and a paper/film laminate.
B14. The package of any one of Examples B1 to B13, wherein the package material has a basis weight in the range of 50 gsm to 100 gsm or 60 gsm to 90 gsm.
B15. The package of any one of Examples B1 to B14, wherein each of the plurality of absorbent articles (10) is compressed.
B16. The package of any one of Examples B1 to B15, wherein each of the plurality of absorbent articles (10) is in open form.
B17. The package of any one of Examples B1 to B16 comprising a plurality of absorbent articles (10) in the form of diapers and/ or pants according to any one of Examples A1 to A17.
C1. A package (1) comprising a plurality of absorbent articles (10) in the form of diapers and/ or pants,
   the package (1) comprising a flexible package material,
   wherein each of the plurality of absorbent articles (10) comprises
      a liquid permeable topsheet (26),
      a liquid impermeable backsheet (28), and
      an absorbent core (30) at least partially positioned between the topsheet (26) and the backsheet (28);
      a front waist region (12), a back waist region (16), a crotch region (14) located between the front waist region (12) and the back waist region (16);
      a central longitudinal axis (50);
      a first fold (400) extending transversely across the crotch region (14) along a first fold line, which is substantially perpendicular to the central longitudinal axis (50); and
      a second fold (500) extending transversely across the front and back waist regions (12, 16) along a second fold line, which is substantially perpendicular to the central longitudinal axis (50);
   wherein the package (1) has a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm according to the Package Compression Test detailed herein.
C2. The package of Example C1, wherein the package (1) has a Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm according to the Package Compression Test detailed herein.
C3. The package of any one of Examples C1 or C2, wherein the package (1) has a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm according to the Package Compression Test detailed herein.
C4. The package of any one of Examples C1 to C3, wherein the package (1) has a Package Out-of-Stack Compression Deflection Differential at 5.0 psi of at most 8.0 mm, preferably at most 7.0 mm, more preferably at most 6.0 mm, even more preferably at most 5.0 mm, most preferably at most 4.0 mm or even at most 3.0 mm according to the Package Compression Test detailed herein.
C5. The package of any one of Examples C1 to C4, wherein the package (1) has a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi according to the Package Compression Test detailed herein.
C6. The package of any one of Examples C1 to C5, wherein the package (1) has a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi according to the Package Compression Test detailed herein.
C7. The package of any one of Examples C1 to C6, wherein the package (1) has a Package In-Stack Waist Compression Deflection Factor of at most 4.25 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi according to the Package Compression Test detailed herein.
C8. The package of any one of Examples C1 to C7, wherein the package (1) has a Package In-Stack Compression Deflection Factor Differential of at most 1.50 mm/ psi, preferably at most 1.25 mm/ psi, more preferably at most 1.00 mm/ psi, even more preferably at most 0.75 mm/ psi, most preferably at most 0.50 mm/ psi or even at most 0.25 mm/ psi according to the Package Compression Test detailed herein.
C9. The package of any one of Examples C1 to C8, wherein the package (1) has a Package In-Stack Waist Compression Deflection at 0.6 psi of at most 2.75 mm, preferably at most 2.50 mm, more preferably at most 2.25 mm, even more preferably at most 2.00 mm, most preferably at most 1.75 mm or even at most 1.50 mm according to the Package Compression Test detailed herein.
C10. The package of any one of Examples C1 to C9, wherein the package (1) has a Package In-Stack Compression Deflection Differential at 0.6 psi of at most 1.00 mm, preferably at most 0.80 mm, more preferably at most 0.60 mm, even more preferably at most 0.40 mm, most preferably at most 0.30 mm or even at most 0.20 mm according to the Package Compression Test detailed herein.
C11. The package of any one of Examples C1 to C10, wherein the package has an in-bag stack height of from 70 mm to 110 mm, according to the In-Bag Stack Height Test detailed herein.
C12. The package of any one of Examples C1 to C11, wherein the package has a Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein.
C13. The package of any of Examples C1 to C12, wherein the flexible package material comprises one or more materials selected from the group consisting of polymeric film, paper, paper based material, paper with one or more barrier layers, and a paper/film laminate.
C14. The package of any one of Examples C1 to C13, wherein the package material has a basis weight in the range of 50 gsm to 100 gsm or 60 gsm to 90 gsm.
C15. The package of any one of Examples C1 to C14, wherein each of the plurality of absorbent articles (10) is compressed.
C16. The package of any one of Examples C1 to C15, wherein each of the plurality of absorbent articles (10) is in open form.
C17. The package of any one of Examples C1 to C16 comprising a plurality of absorbent articles (10) in the form of diapers and/ or pants according to any one of Examples A1 to A17.
D1.A package (1) comprising a plurality of absorbent articles (10) in the form of diapers and/ or pants,
   the package (1) comprising a flexible package material,
   wherein each of the plurality of absorbent articles (10) comprises
      a liquid permeable topsheet (26),
      a liquid impermeable backsheet (28), and
      an absorbent core (30) at least partially positioned between the topsheet (26) and the backsheet (28);
      a front waist region (12), a back waist region (16), a crotch region (14) located between the front waist region (12) and the back waist region (16);
      a central longitudinal axis (50);
      a first fold (400) extending transversely across the crotch region (14) along a first fold line, which is substantially perpendicular to the central longitudinal axis (50); and
      a second fold (500) extending transversely across the front and back waist regions (12, 16) along a second fold line, which is substantially perpendicular to the central longitudinal axis (50);
   wherein the package (1) has a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm according to the Package Compression Test detailed herein.
D2.The package of Example D1, wherein the package (1) has a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm according to the Package Compression Test detailed herein.
D3. The package of any one of Examples D1 or D2, wherein the package (1) has a Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm according to the Package Compression Test detailed herein.
D4.The package of any one of Examples D1 to D3, wherein the package (1) has a Package Out-of-Stack Compression Deflection Differential at 5.0 psi of at most 8.0 mm, preferably at most 7.0 mm, more preferably at most 6.0 mm, even more preferably at most 5.0 mm, most preferably at most 4.0 mm or even at most 3.0 mm according to the Package Compression Test detailed herein.
D5. The package of any one of Examples D1 to D4, wherein the package (1) has a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi according to the Package Compression Test detailed herein.
D6.The package of any one of Examples D1 to D5, wherein the package (1) has a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi according to the Package Compression Test detailed herein.
D7. The package of any one of Examples D1 to D6, wherein the package (1) has a Package In-Stack Waist Compression Deflection Factor of at most 4.25 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi according to the Package Compression Test detailed herein.
D8.The package of any one of Examples D1 to D7, wherein the package (1) has a Package In-Stack Compression Deflection Factor Differential of at most 1.50 mm/ psi, preferably at most 1.25 mm/ psi, more preferably at most 1.00 mm/ psi, even more preferably at most 0.75 mm/ psi, most preferably at most 0.50 mm/ psi or even at most 0.25 mm/ psi according to the Package Compression Test detailed herein.
D9.The package of any one of Examples D1 to D8, wherein the package (1) has a Package In-Stack Waist Compression Deflection at 0.6 psi of at most 2.75 mm, preferably at most 2.50 mm, more preferably at most 2.25 mm, even more preferably at most 2.00 mm, most preferably at most 1.75 mm or even at most 1.50 mm according to the Package Compression Test detailed herein.
D10. The package of any one of Examples D1 to D9, wherein the package (1) has a Package In-Stack Compression Deflection Differential at 0.6 psi of at most 1.00 mm, preferably at most 0.80 mm, more preferably at most 0.60 mm, even more preferably at most 0.40 mm, most preferably at most 0.30 mm or even at most 0.20 mm according to the Package Compression Test detailed herein.
D11. The package of any one of Examples D1 to D10, wherein the package has an in-bag stack height of from 70 mm to 110 mm, according to the In-Bag Stack Height Test detailed herein.
D12. The package of any one of Examples D1 to D11, wherein the package has a Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein.
D13. The package of any of Examples D1 to D12, wherein the flexible package material comprises one or more materials selected from the group consisting of polymeric film, paper, paper based material, paper with one or more barrier layers, and a paper/film laminate.
D14. The package of any one of Examples D1 to D13, wherein the package material has a basis weight in the range of 50 gsm to 100 gsm or 60 gsm to 90 gsm.
D15. The package of any one of Examples D1 to D14, wherein each of the plurality of absorbent articles (10) is compressed.
D16. The package of any one of Examples D1 to D15, wherein each of the plurality of absorbent articles (10) is in open form.
D17. The package of any one of Examples D1 to D16 comprising a plurality of absorbent articles (10) in the form of diapers and/ or pants according to any one of Examples A1 to A17.
E1. A package (1) comprising a plurality of absorbent articles (10) in the form of diapers and/ or pants,
   the package (1) comprising a flexible package material,
   wherein each of the plurality of absorbent articles (10) comprises
      a liquid permeable topsheet (26),
      a liquid impermeable backsheet (28), and
      an absorbent core (30) at least partially positioned between the topsheet (26) and the backsheet (28);
      a front waist region (12), a back waist region (16), a crotch region (14) located between the front waist region (12) and the back waist region (16);
      a central longitudinal axis (50);
      a first fold (400) extending transversely across the crotch region (14) along a first fold line, which is substantially perpendicular to the central longitudinal axis (50); and
      a second fold (500) extending transversely across the front and back waist regions (12, 16) along a second fold line, which is substantially perpendicular to the central longitudinal axis (50);
   wherein the package (1) has a Package Out-of-Stack Compression Deflection Differential at 5.0 psi of at most 8.0 mm, preferably at most 7.0 mm, more preferably at most 6.0 mm, even more preferably at most 5.0 mm, most preferably at most 4.0 mm or even at most 3.0 mm according to the Package Compression Test detailed herein.
E2. The package of Example E1, wherein the package (1) has a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm according to the Package Compression Test detailed herein.
E3. The package of any one of Examples E1 or E2, wherein the package (1) has a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm according to the Package Compression Test detailed herein.
E4. The package of any one of Examples E1 to E3, wherein the package (1) has a Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm according to the Package Compression Test detailed herein.
E5. The package of any one of Examples E1 to E4, wherein the package (1) has a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi according to the Package Compression Test detailed herein.
E6. The package of any one of Examples E1 to E5, wherein the package (1) has a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi according to the Package Compression Test detailed herein.
E7. The package of any one of Examples E1 to E6, wherein the package (1) has a Package In-Stack Waist Compression Deflection Factor of at most 4.25 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi according to the Package Compression Test detailed herein.
E8. The package of any one of Examples E1 to E7, wherein the package (1) has a Package In-Stack Compression Deflection Factor Differential of at most 1.50 mm/ psi, preferably at most 1.25 mm/ psi, more preferably at most 1.00 mm/ psi, even more preferably at most 0.75 mm/ psi, most preferably at most 0.50 mm/ psi or even at most 0.25 mm/ psi according to the Package Compression Test detailed herein.
E9. The package of any one of Examples E1 to E8, wherein the package (1) has a Package In-Stack Waist Compression Deflection at 0.6 psi of at most 2.75 mm, preferably at most 2.50 mm, more preferably at most 2.25 mm, even more preferably at most 2.00 mm, most preferably at most 1.75 mm or even at most 1.50 mm according to the Package Compression Test detailed herein.
E10. The package of any one of Examples E1 to E9, wherein the package (1) has a Package In-Stack Compression Deflection Differential at 0.6 psi of at most 1.00 mm, preferably at most 0.80 mm, more preferably at most 0.60 mm, even more preferably at most 0.40 mm, most preferably at most 0.30 mm or even at most 0.20 mm according to the Package Compression Test detailed herein.
E11. The package of any one of Examples E1 to E10, wherein the package has an in-bag stack height of from 70 mm to 110 mm, according to the In-Bag Stack Height Test detailed herein.
E12. The package of any one of Examples E1 to E11, wherein the package has a Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein.
E13. The package of any of Examples E1 to E12, wherein the flexible package material comprises one or more materials selected from the group consisting of polymeric film, paper, paper based material, paper with one or more barrier layers, and a paper/film laminate.
E14. The package of any one of Examples E1 to E13, wherein the package material has a basis weight in the range of 50 gsm to 100 gsm or 60 gsm to 90 gsm.
E15. The package of any one of Examples E1 to E14, wherein each of the plurality of absorbent articles (10) is compressed.
E16. The package of any one of Examples E1 to E15, wherein each of the plurality of absorbent articles (10) is in open form.
E17. The package of any one of Examples E1 to E16 comprising a plurality of absorbent articles (10) in the form of diapers and/ or pants according to any one of Examples A1 to A17.
F1. A package (1) comprising a plurality of absorbent articles (10) in the form of diapers and/ or pants,
   the package (1) comprising a flexible package material,
   wherein each of the plurality of absorbent articles (10) comprises
      a liquid permeable topsheet (26),
      a liquid impermeable backsheet (28), and
      an absorbent core (30) at least partially positioned between the topsheet (26) and the backsheet (28);
      a front waist region (12), a back waist region (16), a crotch region (14) located between the front waist region (12) and the back waist region (16);
      a central longitudinal axis (50);
      a first fold (400) extending transversely across the crotch region (14) along a first fold line, which is substantially perpendicular to the central longitudinal axis (50); and
      a second fold (500) extending transversely across the front and back waist regions (12, 16) along a second fold line, which is substantially perpendicular to the central longitudinal axis (50);
   wherein the package (1) has a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi according to the Package Compression Test detailed herein.
F2. The package of Example F1, wherein the package (1) has a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm according to the Package Compression Test detailed herein.
F3. The package of any one of Examples F1 or F2, wherein the package (1) has a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm according to the Package Compression Test detailed herein.
F4. The package of any one of Examples F1 to F3, wherein the package (1) has a Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm according to the Package Compression Test detailed herein.
F5. The package of any one of Examples F1 to F4, wherein the package (1) has a Package Out-of-Stack Compression Deflection Differential at 5.0 psi of at most 8.0 mm, preferably at most 7.0 mm, more preferably at most 6.0 mm, even more preferably at most 5.0 mm, most preferably at most 4.0 mm or even at most 3.0 mm according to the Package Compression Test detailed herein.
F6. The package of any one of Examples F1 to F5, wherein the package (1) has a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi according to the Package Compression Test detailed herein.
F7. The package of any one of Examples F1 to F6, wherein the package (1) has a Package In-Stack Waist Compression Deflection Factor of at most 4.25 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi according to the Package Compression Test detailed herein.
F8. The package of any one of Examples F1 to F7, wherein the package (1) has a Package In-Stack Compression Deflection Factor Differential of at most 1.50 mm/ psi, preferably at most 1.25 mm/ psi, more preferably at most 1.00 mm/ psi, even more preferably at most 0.75 mm/ psi, most preferably at most 0.50 mm/ psi or even at most 0.25 mm/ psi according to the Package Compression Test detailed herein.
F9. The package of any one of Examples F1 to F8, wherein the package (1) has a Package In-Stack Waist Compression Deflection at 0.6 psi of at most 2.75 mm, preferably at most 2.50 mm, more preferably at most 2.25 mm, even more preferably at most 2.00 mm, most preferably at most 1.75 mm or even at most 1.50 mm according to the Package Compression Test detailed herein.
F10. The package of any one of Examples F1 to F9, wherein the package (1) has a Package In-Stack Compression Deflection Differential at 0.6 psi of at most 1.00 mm, preferably at most 0.80 mm, more preferably at most 0.60 mm, even more preferably at most 0.40 mm, most preferably at most 0.30 mm or even at most 0.20 mm according to the Package Compression Test detailed herein.
F11. The package of any one of Examples F1 to F10, wherein the package has an in-bag stack height of from 70 mm to 110 mm, according to the In-Bag Stack Height Test detailed herein.
F12. The package of any one of Examples F1 to F11, wherein the package has a Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein.
F13. The package of any of Examples F1 to F12, wherein the flexible package material comprises one or more materials selected from the group consisting of polymeric film, paper, paper based material, paper with one or more barrier layers, and a paper/film laminate.
F14. The package of any one of Examples F1 to F13, wherein the package material has a basis weight in the range of 50 gsm to 100 gsm or 60 gsm to 90 gsm.
F15. The package of any one of Examples F1 to F14, wherein each of the plurality of absorbent articles (10) is compressed.
F16. The package of any one of Examples F1 to F15, wherein each of the plurality of absorbent articles (10) is in open form.
F17. The package of any one of Examples F1 to F16 comprising a plurality of absorbent articles (10) in the form of diapers and/ or pants according to any one of Examples A1 to A17.
G1.A package (1) comprising a plurality of absorbent articles (10) in the form of diapers and/ or pants,
   the package (1) comprising a flexible package material,
   wherein each of the plurality of absorbent articles (10) comprises
      a liquid permeable topsheet (26),
      a liquid impermeable backsheet (28), and
      an absorbent core (30) at least partially positioned between the topsheet (26) and the backsheet (28);
      a front waist region (12), a back waist region (16), a crotch region (14) located between the front waist region (12) and the back waist region (16);
      a central longitudinal axis (50);
      a first fold (400) extending transversely across the crotch region (14) along a first fold line, which is substantially perpendicular to the central longitudinal axis (50); and
      a second fold (500) extending transversely across the front and back waist regions (12, 16) along a second fold line, which is substantially perpendicular to the central longitudinal axis (50);
   wherein the package (1) has a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi according to the Package Compression Test detailed herein.
G2.The package of Example G1, wherein the package (1) has a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm according to the Package Compression Test detailed herein.
G3. The package of any one of Examples G1 or G2, wherein the package (1) has a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm according to the Package Compression Test detailed herein.
G4. The package of any one of Examples G1 to G3, wherein the package (1) has a Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm according to the Package Compression Test detailed herein.
G5. The package of any one of Examples G1 to G4, wherein the package (1) has a Package Out-of-Stack Compression Deflection Differential at 5.0 psi of at most 8.0 mm, preferably at most 7.0 mm, more preferably at most 6.0 mm, even more preferably at most 5.0 mm, most preferably at most 4.0 mm or even at most 3.0 mm according to the Package Compression Test detailed herein.
G6.The package of any one of Examples G1 to G5, wherein the package (1) has a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi according to the Package Compression Test detailed herein.
G7. The package of any one of Examples G1 to G6, wherein the package (1) has a Package In-Stack Waist Compression Deflection Factor of at most 4.25 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi according to the Package Compression Test detailed herein.
G8. The package of any one of Examples G1 to G7, wherein the package (1) has a Package In-Stack Compression Deflection Factor Differential of at most 1.50 mm/ psi, preferably at most 1.25 mm/ psi, more preferably at most 1.00 mm/ psi, even more preferably at most 0.75 mm/ psi, most preferably at most 0.50 mm/ psi or even at most 0.25 mm/ psi according to the Package Compression Test detailed herein.
G9. The package of any one of Examples G1 to G8, wherein the package (1) has a Package In-Stack Waist Compression Deflection at 0.6 psi of at most 2.75 mm, preferably at most 2.50 mm, more preferably at most 2.25 mm, even more preferably at most 2.00 mm, most preferably at most 1.75 mm or even at most 1.50 mm according to the Package Compression Test detailed herein.
G10. The package of any one of Examples G1 to G9, wherein the package (1) has a Package In-Stack Compression Deflection Differential at 0.6 psi of at most 1.00 mm, preferably at most 0.80 mm, more preferably at most 0.60 mm, even more preferably at most 0.40 mm, most preferably at most 0.30 mm or even at most 0.20 mm according to the Package Compression Test detailed herein.
G11. The package of any one of Examples G1 to G10, wherein the package has an in-bag stack height of from 70 mm to 110 mm, according to the In-Bag Stack Height Test detailed herein.
G12. The package of any one of Examples G1 to G11, wherein the package has a Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein.
G13. The package of any of Examples G1 to G12, wherein the flexible package material comprises one or more materials selected from the group consisting of polymeric film, paper, paper based material, paper with one or more barrier layers, and a paper/film laminate.
G14. The package of any one of Examples G1 to G13, wherein the package material has a basis weight in the range of 50 gsm to 100 gsm or 60 gsm to 90 gsm.
G15. The package of any one of Examples G1 to G14, wherein each of the plurality of absorbent articles (10) is compressed.
G16. The package of any one of Examples G1 to G15, wherein each of the plurality of absorbent articles (10) is in open form.
G17. The package of any one of Examples G1 to G16 comprising a plurality of absorbent articles (10) in the form of diapers and/ or pants according to any one of Examples A1 to A17.
H1. A package (1) comprising a plurality of absorbent articles (10) in the form of diapers and/ or pants,
   the package (1) comprising a flexible package material,
   wherein each of the plurality of absorbent articles (10) comprises
      a liquid permeable topsheet (26),
      a liquid impermeable backsheet (28), and
      an absorbent core (30) at least partially positioned between the topsheet (26) and the backsheet (28);
      a front waist region (12), a back waist region (16), a crotch region (14) located between the front waist region (12) and the back waist region (16);
      a central longitudinal axis (50);
      a first fold (400) extending transversely across the crotch region (14) along a first fold line, which is substantially perpendicular to the central longitudinal axis (50); and
      a second fold (500) extending transversely across the front and back waist regions (12, 16) along a second fold line, which is substantially perpendicular to the central longitudinal axis (50);
   wherein the package (1) has a Package In-Stack Waist Compression Deflection Factor of at most 4.25 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi according to the Package Compression Test detailed herein.
H2.The package of Example H1, wherein the package (1) has a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm according to the Package Compression Test detailed herein.
H3. The package of any one of Examples H1 or H2, wherein the package (1) has a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm according to the Package Compression Test detailed herein.
H4. The package of any one of Examples H1 to H3, wherein the package (1) has a Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm according to the Package Compression Test detailed herein.
H5. The package of any one of Examples H1 to H4, wherein the package (1) has a Package Out-of-Stack Compression Deflection Differential at 5.0 psi of at most 8.0 mm, preferably at most 7.0 mm, more preferably at most 6.0 mm, even more preferably at most 5.0 mm, most preferably at most 4.0 mm or even at most 3.0 mm according to the Package Compression Test detailed herein.
H6.The package of any one of Examples H1 to H5, wherein the package (1) has a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi according to the Package Compression Test detailed herein.
H7. The package of any one of Examples H1 to H6, wherein the package (1) has a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi according to the Package Compression Test detailed herein.
H8. The package of any one of Examples H1 to H7, wherein the package (1) has a Package In-Stack Compression Deflection Factor Differential of at most 1.50 mm/ psi, preferably at most 1.25 mm/ psi, more preferably at most 1.00 mm/ psi, even more preferably at most 0.75 mm/ psi, most preferably at most 0.50 mm/ psi or even at most 0.25 mm/ psi according to the Package Compression Test detailed herein.
H9. The package of any one of Examples H1 to H8, wherein the package (1) has a Package In-Stack Waist Compression Deflection at 0.6 psi of at most 2.75 mm, preferably at most 2.50 mm, more preferably at most 2.25 mm, even more preferably at most 2.00 mm, most preferably at most 1.75 mm or even at most 1.50 mm according to the Package Compression Test detailed herein.
H10. The package of any one of Examples H1 to H9, wherein the package (1) has a Package In-Stack Compression Deflection Differential at 0.6 psi of at most 1.00 mm, preferably at most 0.80 mm, more preferably at most 0.60 mm, even more preferably at most 0.40 mm, most preferably at most 0.30 mm or even at most 0.20 mm according to the Package Compression Test detailed herein.
H11. The package of any one of Examples H1 to H10, wherein the package has an in-bag stack height of from 70 mm to 110 mm, according to the In-Bag Stack Height Test detailed herein.
H12. The package of any one of Examples H1 to H11, wherein the package has a Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein.
H13. The package of any of Examples H1 to H12, wherein the flexible package material comprises one or more materials selected from the group consisting of polymeric film, paper, paper based material, paper with one or more barrier layers, and a paper/film laminate.
H14. The package of any one of Examples H1 to H13, wherein the package material has a basis weight in the range of 50 gsm to 100 gsm or 60 gsm to 90 gsm.
H15. The package of any one of Examples H1 to H14, wherein each of the plurality of absorbent articles (10) is compressed.
H16. The package of any one of Examples H1 to H15, wherein each of the plurality of absorbent articles (10) is in open form.
H17. The package of any one of Examples H1 to H16 comprising a plurality of absorbent articles (10) in the form of diapers and/ or pants according to any one of Examples A1 to A17.
I1. A package (1) comprising a plurality of absorbent articles (10) in the form of diapers and/ or pants,
   the package (1) comprising a flexible package material,
   wherein each of the plurality of absorbent articles (10) comprises
      a liquid permeable topsheet (26),
      a liquid impermeable backsheet (28), and
      an absorbent core (30) at least partially positioned between the topsheet (26) and the backsheet (28);
      a front waist region (12), a back waist region (16), a crotch region (14) located between the front waist region (12) and the back waist region (16);
      a central longitudinal axis (50);
      a first fold (400) extending transversely across the crotch region (14) along a first fold line, which is substantially perpendicular to the central longitudinal axis (50); and
      a second fold (500) extending transversely across the front and back waist regions (12, 16) along a second fold line, which is substantially perpendicular to the central longitudinal axis (50);
   wherein the package (1) has a Package In-Stack Compression Deflection Factor Differential of at most 1.50 mm/ psi, preferably at most 1.25 mm/ psi, more preferably at most 1.00 mm/ psi, even more preferably at most 0.75 mm/ psi, most preferably at most 0.50 mm/ psi or even at most 0.25 mm/ psi according to the Package Compression Test detailed herein.
I2. The package of Example I1, wherein the package (1) has a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm according to the Package Compression Test detailed herein.
I3. The package of any one of Examples I1 or I2, wherein the package (1) has a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm according to the Package Compression Test detailed herein.
I4. The package of any one of Examples I1 to I3, wherein the package (1) has a Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm according to the Package Compression Test detailed herein.
I5. The package of any one of Examples I1 to I4, wherein the package (1) has a Package Out-of-Stack Compression Deflection Differential at 5.0 psi of at most 8.0 mm, preferably at most 7.0 mm, more preferably at most 6.0 mm, even more preferably at most 5.0 mm, most preferably at most 4.0 mm or even at most 3.0 mm according to the Package Compression Test detailed herein.
I6. The package of any one of Examples I1 to I5, wherein the package (1) has a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi according to the Package Compression Test detailed herein.
I7. The package of any one of Examples I1 to I6, wherein the package (1) has a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi according to the Package Compression Test detailed herein.
I8. The package of any one of Examples I1 to I7, wherein the package (1) has a Package In-Stack Waist Compression Deflection Factor of at most 4.25 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi according to the Package Compression Test detailed herein.
I9. The package of any one of Examples I1 to I8, wherein the package (1) has a Package In-Stack Waist Compression Deflection at 0.6 psi of at most 2.75 mm, preferably at most 2.50 mm, more preferably at most 2.25 mm, even more preferably at most 2.00 mm, most preferably at most 1.75 mm or even at most 1.50 mm according to the Package Compression Test detailed herein.
I10. The package of any one of Examples I1 to I9, wherein the package (1) has a Package In-Stack Compression Deflection Differential at 0.6 psi of at most 1.00 mm, preferably at most 0.80 mm, more preferably at most 0.60 mm, even more preferably at most 0.40 mm, most preferably at most 0.30 mm or even at most 0.20 mm according to the Package Compression Test detailed herein.
I11. The package of any one of Examples I1 to I10, wherein the package has an in-bag stack height of from 70 mm to 110 mm, according to the In-Bag Stack Height Test detailed herein.
I12. The package of any one of Examples I1 to I11, wherein the package has a Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein.
I13. The package of any of Examples I1 to I12, wherein the flexible package material comprises one or more materials selected from the group consisting of polymeric film, paper, paper based material, paper with one or more barrier layers, and a paper/film laminate.
I14. The package of any one of Examples I1 to I13, wherein the package material has a basis weight in the range of 50 gsm to 100 gsm or 60 gsm to 90 gsm.
I15. The package of any one of Examples I1 to I14, wherein each of the plurality of absorbent articles (10) is compressed.
I16. The package of any one of Examples I1 to I15, wherein each of the plurality of absorbent articles (10) is in open form.
I17. The package of any one of Examples I1 to I16 comprising a plurality of absorbent articles (10) in the form of diapers and/ or pants according to any one of Examples A1 to A17.
K1. A package (1) comprising a plurality of absorbent articles (10) in the form of diapers and/ or pants,
   the package (1) comprising a flexible package material,
   wherein each of the plurality of absorbent articles (10) comprises
      a liquid permeable topsheet (26),
      a liquid impermeable backsheet (28), and
      an absorbent core (30) at least partially positioned between the topsheet (26) and the backsheet (28);
      a front waist region (12), a back waist region (16), a crotch region (14) located between the front waist region (12) and the back waist region (16);
      a central longitudinal axis (50);
      a first fold (400) extending transversely across the crotch region (14) along a first fold line, which is substantially perpendicular to the central longitudinal axis (50); and
      a second fold (500) extending transversely across the front and back waist regions (12, 16) along a second fold line, which is substantially perpendicular to the central longitudinal axis (50);
   wherein the package (1) exhibits one or more of the properties a) to j) according to the Package Compression Test detailed herein:
   a) a Package In-Stack Compression Deflection Factor Differential of at most 1.50 mm/ psi, preferably at most 1.25 mm/ psi, more preferably at most 1.00 mm/ psi, even more preferably at most 0.75 mm/ psi, most preferably at most 0.50 mm/ psi or even at most 0.25 mm/ psi;
   b) a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm;
   c) a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm;
   d) a Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm;
   e) a Package Out-of-Stack Compression Deflection Differential at 5.0 psi of at most 8.0 mm, preferably at most 7.0 mm, more preferably at most 6.0 mm, even more preferably at most 5.0 mm, most preferably at most 4.0 mm or even at most 3.0 mm;
   f) a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi;
   g) a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi ;
   h) a Package In-Stack Waist Compression Deflection Factor of at most 4.25 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi;
   i) a Package In-Stack Waist Compression Deflection at 0.6 psi of at most 2.75 mm, preferably at most 2.50 mm, more preferably at most 2.25 mm, even more preferably at most 2.00 mm, most preferably at most 1.75 mm or even at most 1.50 mm;
   j) a Package In-Stack Compression Deflection Differential at 0.6 psi of at most 1.00 mm, preferably at most 0.80 mm, more preferably at most 0.60 mm, even more preferably at most 0.40 mm, most preferably at most 0.30 mm or even at most 0.20 mm.
K2. The package of Example K1, wherein the package exhibits at least two of the properties a) to j) according to the Package Compression Test detailed herein.
K3. The package of Examples K1 or K2, wherein the package exhibits at least three, at least four, at least five or even at least six of the properties a) to j) according to the Package Compression Test detailed herein.
K4. The package of Examples K1 to K3, wherein the package exhibits all of the properties a) to j) according to the Package Compression Test detailed herein.
K5. The package of any one of Examples K1 to K4, wherein the package has an in-bag stack height of from 70 mm to 110 mm, according to the In-Bag Stack Height Test detailed herein.
K6. The package of any one of Examples K1 to K5, wherein the package has a Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein.
K7. The package of any of Examples K1 to K6, wherein the flexible package material comprises one or more materials selected from the group consisting of polymeric film, paper, paper based material, paper with one or more barrier layers, and a paper/film laminate.
K8. The package of any one of Examples K1 to K7, wherein the package material has a basis weight in the range of 50 gsm to 100 gsm or 60 gsm to 90 gsm.
K9. The package of any one of Examples K1 to K8, wherein each of the plurality of absorbent articles (10) is compressed.
K10. The package of any one of Examples K1 to K9, wherein each of the plurality of absorbent articles (10) is in open form.
K11. The package of any one of Examples K1 to K10 comprising a plurality of absorbent articles (10) in the form of diapers and/ or pants according to any one of Examples A1 to A17.
L1. A package (1) comprising a plurality of absorbent articles (10) in the form of diapers and/ or pants,
   the package (1) comprising a flexible package material,
   wherein each of the plurality of absorbent articles (10) comprises
      a liquid permeable topsheet (26),
      a liquid impermeable backsheet (28), and
      an absorbent core (30) at least partially positioned between the topsheet (26) and the backsheet (28);
      a front waist region (12), a back waist region (16), a crotch region (14) located between the front waist region (12) and the back waist region (16);
      a central longitudinal axis (50);
      a first fold (400) extending transversely across the crotch region (14) along a first fold line, which is substantially perpendicular to the central longitudinal axis (50); and
      a second fold (500) extending transversely across the front and back waist regions (12, 16) along a second fold line, which is substantially perpendicular to the central longitudinal axis (50);
   wherein the package (1) exhibits one or more of the properties a) to f) and exhibits one or more of the properties g) to j) according to the Package Compression Test detailed herein:
   a) a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm;
   b) a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm;
   c) a Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm;
   d) a Package Out-of-Stack Compression Deflection Differential at 5.0 psi of at most 8.0 mm, preferably at most 7.0 mm, more preferably at most 6.0 mm, even more preferably at most 5.0 mm, most preferably at most 4.0 mm or even at most 3.0 mm;
   e) a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi;
   f) a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi;
   g) a Package In-Stack Compression Deflection Factor Differential of at most 1.50 mm/ psi, preferably at most 1.25 mm/ psi, more preferably at most 1.00 mm/ psi, even more preferably at most 0.75 mm/ psi, most preferably at most 0.50 mm/ psi or even at most 0.25 mm/ psi;
   h) a Package In-Stack Waist Compression Deflection Factor of at most 4.25 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi;
   i) a Package In-Stack Waist Compression Deflection at 0.6 psi of at most 2.75 mm, preferably at most 2.50 mm, more preferably at most 2.25 mm, even more preferably at most 2.00 mm, most preferably at most 1.75 mm or even at most 1.50 mm;
   j) a Package In-Stack Compression Deflection Differential at 0.6 psi of at most 1.00 mm, preferably at most 0.80 mm, more preferably at most 0.60 mm, even more preferably at most 0.40 mm, most preferably at most 0.30 mm or even at most 0.20 mm.
L2. The package of Example L1, wherein the package exhibits at least two of the properties a) to f) according to the Package Compression Test detailed herein.
L3. The package of Examples L1 or L2, wherein the package exhibits at least three, at least four, at least five or even at least six of the properties a) to f) according to the Package Compression Test detailed herein.
L4. The package of Examples L1 to L3, wherein the package exhibits at least two of the properties g) to j) according to the Package Compression Test detailed herein.
L5. The package of Examples L1 to L4, wherein the package exhibits at least three, at least four, at least five or even at least six of the properties g) to j) according to the Package Compression Test detailed herein.
L6. The package of Examples L1 to L5, wherein the package exhibits all of the properties a) to j) according to the Package Compression Test detailed herein.
L7. The package of any one of Examples L1 to L6, wherein the package has an in-bag stack height of from 70 mm to 110 mm, according to the In-Bag Stack Height Test detailed herein.
L8. The package of any one of Examples L1 to L7, wherein the package has a Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein.
L9. The package of any of Examples L1 to L8, wherein the flexible package material comprises one or more materials selected from the group consisting of polymeric film, paper, paper based material, paper with one or more barrier layers, and a paper/film laminate.
L10. The package of any one of Examples L1 to L9, wherein the package material has a basis weight in the range of 50 gsm to 100 gsm or 60 gsm to 90 gsm.
L11. The package of any one of Examples L1 to L10, wherein each of the plurality of absorbent articles (10) is compressed.
L12. The package of any one of Examples L1 to L11, wherein each of the plurality of absorbent articles (10) is in open form.
L 13. The package of any one of Examples L1 to L12 comprising a plurality of absorbent articles (10) in the form of diapers and/ or pants according to any one of Examples A1 to A17.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A package (1) comprising a plurality of absorbent articles (10) in the form of diapers,
the package (1) comprising a flexible package material,
wherein each of the plurality of absorbent articles (10) is in open form and comprises
a liquid permeable topsheet (26),
a liquid impermeable backsheet (28), and
an absorbent core (30) at least partially positioned between the topsheet (26) and the backsheet (28);
a front waist region (12), a back waist region (16), a crotch region (14) located between the front waist region (12) and the back waist region (16);
a central longitudinal axis (50);
a first fold (400) extending transversely across the crotch region (14) along a first fold line, which is substantially perpendicular to the central longitudinal axis (50); and
a second fold (500) extending transversely across the front and back waist regions (12, 16) along a second fold line, which is substantially perpendicular to the central longitudinal axis (50);
wherein the package (1) has a Package Out-of-Stack Compression Deflection Differential at 0.6 psi of at most 3.0 mm, preferably at most 2.5 mm, more preferably at most 2.0 mm, even more preferably at most 1.5 mm, most preferably at most 1.0 mm or even at most 0.5 mm according to the Package Compression Test detailed herein.

2. The package of any one of the preceding claims, wherein the package (1) has Package Out-of-Stack Waist Compression Deflection at 0.6 psi of at most 4.5 mm, preferably at most 4.0 mm, more preferably at most 3.5 mm, even more preferably at most 3.0 mm, most preferably at most 2.5 mm or even at most 2.0 mm according to the Package Compression Test detailed herein.

3. The package of any one of the preceding claims, wherein the package (1) has a Package Out-of-Stack Waist Compression Deflection at 5.0 psi of at most 15.0 mm, preferably at most 12.5 mm, more preferably at most 10.0 mm, even more preferably at most 9.0 mm, most preferably at most 8.5 mm or even at most 8.0 mm according to the Package Compression Test detailed herein.

4. The package of any one of the preceding claims, wherein the package (1) has a Package Out-of-Stack Waist Compression Deflection Factor of at most 2.25 mm/ psi, preferably at most 2.00 mm/ psi, more preferably at most 1.75 mm/ psi, even more preferably at most 1.50 mm/ psi, most preferably at most 1.25 mm/ psi or even at most 1.00 mm/ psi according to the Package Compression Test detailed herein.

5. The package of any one of the preceding claims, wherein the package (1) has a Package Out-of-Stack Compression Deflection Factor Differential of at most 0.80 mm/ psi, preferably at most 0.50 mm/ psi, more preferably at most 0.40 mm/ psi, even more preferably at most 0.30 mm/ psi, most preferably at most 0.20 mm/ psi or even at most 0.10 mm/ psi according to the Package Compression Test detailed herein.

6. The package of any one of the preceding claims, wherein the package (1) has a Package In-Stack Waist Compression Deflection Factor of at most 4.25 mm/ psi, preferably at most 4.00 mm/ psi, more preferably at most 3.75 mm/ psi, even more preferably at most 3.50 mm/ psi, most preferably at most 3.25 mm/ psi or even at most 3.00 mm/ psi according to the Package Compression Test detailed herein.

7. The package of any one of the preceding claims, wherein the package (1) has a Package In-Stack Compression Deflection Factor Differential of at most 1.50 mm/ psi, preferably at most 1.25 mm/ psi, more preferably at most 1.00 mm/ psi, even more preferably at most 0.75 mm/ psi, most preferably at most 0.50 mm/ psi or even at most 0.25 mm/ psi according to the Package Compression Test detailed herein.

8. The package of any one of the preceding claims, wherein the package has an in-bag stack height of from 70 mm to 110 mm, according to the In-Bag Stack Height Test detailed herein.

9. The package of any one of the preceding claims, wherein the package has a Package Center of Gravity at from 20% to 49% of the Package Out-of-Stack Length, preferably at from 25% to 48%, more preferably at from 30% to 47%, even more preferably at from 35% to 46%, most preferably at from 37% to 45%, or even from 40% to 44% of the Package Out-of-Stack Length and at from 40% to 60% of the Package In-Stack Length, preferably from 45% to 55%, more preferably from 48 to 52 or even about 50% of the Package In-Stack Length according to the Package Center of Gravity Test detailed herein.

10. The package of any one of the preceding claims, wherein each of the plurality of absorbent articles (10) comprises back ears (42) in the back waist region (16).

11. An absorbent article (10) in the form of diapers comprising
a liquid permeable topsheet (26),
a liquid impermeable backsheet (28), and
an absorbent core (30) at least partially positioned between the topsheet (26) and the backsheet (28);
a front waist region (12), a back waist region (16), a crotch region (14) located between the front waist region (12) and the back waist region (16);
back ears (42) in the back waist region (16);
a central longitudinal axis (50);
a first fold (400) extending transversely across the crotch region (14) along a first fold line, which is substantially perpendicular to the central longitudinal axis (50); and further comprising a second fold (500) extending transversely across the front and back waist regions (12, 16) along a second fold line, which is substantially perpendicular to the central longitudinal axis (50);
wherein the second fold line lies between the first fold line and a front waist edge of the absorbent core (112) in the front waist region (12) and/ or lies between the first fold line and a back waist edge of the absorbent core (116) in the back waist region (16).

12. The absorbent article (10) of claim 11, wherein the absorbent article (10) further comprises an acquisition and distribution system (38) at least partially positioned intermediate the absorbent core (30) and the topsheet (26) and wherein the crotch region (14) at least partially comprises the acquisition and distribution system (38)
and wherein the second fold line
either corresponds to a front waist edge of the acquisition and distribution system (212) or lies between the front waist edge of the acquisition and distribution system (212) and the front waist edge of the absorbent core (112) in the front waist region (12); and
either corresponds to a back waist edge of the acquisition and distribution system (216) or lies between the front waist edge of the acquisition and distribution system (212) and the front waist edge of the absorbent core (112) in the front waist region (12) and/ or lies between the back waist edge of the acquisition and distribution system (216) and the back waist edge of the absorbent core (116) in the back waist region (16).

13. The absorbent article (10) of any one of claims 11 or 12, wherein the absorbent core (30) comprises absorbent material (72) comprising a blend of cellulose fibers and superabsorbent particles; and/ or high internal phase emulsion foam and wherein the absorbent material (72) is non-uniformly distributed at least in the direction of the longitudinal axis (50).

14. The absorbent article (10) of any one of claims 11 to 13, wherein absorbent article (10) the first fold line divides the crotch region (14) into two parts of unequal length in the direction of the central longitudinal axis (50).

15. The absorbent article (10) of claim 14, wherein the first fold line is closer to the front-end edge (18) than to the back-end edge (20) of the absorbent article (10).

16. The absorbent article (10) of any one of claims 10 to 15, wherein the absorbent article (10) further comprises an elastic waistband (36).

17. The package of any one of claims 1 to 10 comprising a plurality of absorbent articles (10) in the form of diapers and/ or pants according to any one of claims 11 to 16.
